(12) **EUROPEAN PATENT SPECIFICATION**

(45) Date of publication and mention
of the grant of the patent:
**17.04.2019 Bulletin 2019/16**

(51) Int Cl.:
**G01N 33/68** (2006.01)

(21) Application number: **15793908.3**

(22) Date of filing: **20.10.2015**

(86) International application number:
**PCT/IB2015/058087**

(87) International publication number:
**WO 2016/063223 (28.04.2016 Gazette 2016/17)**

(54) **METHODS FOR PREDICTING CLINICAL OUTCOMES IN SUBJECTS AFFLICTED WITH ULCERATIVE COLITIS**

VERFAHREN ZUR VORHERSAGE DER KLINISCHEN ERGEBNISSEN IN PATIENTEN MIT COLITIS ULCEROSA

PROCÉDÉ DE PRÉDICTION DES ISSUS CLINIQUES DES PATIENTS ATTEINTS DE RECTOCOLITE HÉMORRAGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.10.2014 US 201462066209 P
02.12.2014 US 201462086512 P
12.05.2015 US 201562160551 P**

(43) Date of publication of application:
**30.08.2017 Bulletin 2017/35**

(73) Proprietor: **Nestec S.A.
1800 Vevey (CH)**

(72) Inventors:
• **SINGH, Sharat
San Diego
California 92121 (US)**
• **JAIN, Anjali
San Diego
California 92129 (US)**

(74) Representative: **J A Kemp
14 South Square
Gray's Inn
London WC1R 5JJ (GB)**

(56) References cited:
**WO-A1-2014/054013    WO-A2-2013/080050
US-A1- 2013 040 835**

• **MICHAEL MACORITTO ET AL: "568 Therapeutic Diagnostic: Identification of Molecular Mechanisms Underlying Ulcerative Colitis in Infliximab Non-Responders at Baseline", GASTROENTEROLOGY, vol. 142, no. 5, 18 April 2012 (2012-04-18), pages S-115, XP055234440, PHILADELPHIA, PA ISSN: 0016-5085, DOI: 10.1016/S0016-5085(12)60433-8**
• **M. K. MAGNUSSON ET AL: "CD25 and TNF receptor II reflect early primary response to infliximab therapy in patients with ulcerative colitis", UNITED EUROPEAN GASTROENTEROLOGY JOURNAL, vol. 1, no. 6, 23 August 2013 (2013-08-23) , pages 467-476, XP055234445, ISSN: 2050-6406, DOI: 10.1177/2050640613502962**
• **GARY TOEDTER ET AL: "Gene Expression Profiling and Response Signatures Associated With Differential Responses to Infliximab Treatment in Ulcerative Colitis", AMERICAN JOURNAL OF GASTROENTEROLOGY, vol. 106, no. 7, 29 March 2011 (2011-03-29), pages 1272-1280, XP055234476, US ISSN: 0002-9270, DOI: 10.1038/ajg.2011.83**
• **RISMO RENATHE ET AL: "Mucosal cytokine gene expression profiles as biomarkers of response to infliximab in ulcerative colitis", SCANDINAVIAN JOURNAL OF GASTROENTEROLOGY, INFORMA HEALTHCARE, UK, vol. 47, no. 5, 10 April 2012 (2012-04-10) , pages 538-547, XP009176535, ISSN: 0036-5521**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 210 027 B1

- ARIJS I ET AL: "Mucosal gene signatures to predict response to infliximab in patients with ulcerative colitis", GUT, BRITISH MEDICAL ASSOCIATION, LONDON, UK, vol. 58, no. 12, 20 August 2009 (2009-08-20), pages 1612-1619, XP002620412, ISSN: 0017-5749, DOI: 10.1136/GUT.2009.178665 [retrieved on 2009-08-20]

- Cole Harris ET AL: "Gene Expression Signatures Predictive of Infliximab Response in IBD", AMERICAN JOURNAL OF GASTROENTEROLOGY, vol. 106, no. s2, 28 October 2011 (2011-10-28), page s459, XP055458552, US ISSN: 0002-9270

**Description**

**BACKGROUND OF THE INVENTION**

Ulcerative colitis (UC) is a disease of the large intestine characterized recurring episodes of inflammation of the mucosal layer of the large intestines which can manifest as chronic diarrhea with cramping, abdominal pain, rectal bleeding, loose discharges of blood, pus, and mucus. A pattern of exacerbations and remissions typifies the clinical course for about 70% of UC patients, although continuous symptoms without remission are present in some patients with UC. Local and systemic complications of UC include arthritis, eye inflammation such as uveitis, skin ulcers, and liver disease. In addition, UC, and especially the long-standing, extensive form of the disease is associated with an increased risk of colon carcinoma.

UC is a diffuse disease that usually extends from the most distal part of the rectum for a variable distance proximally. The term "left-sided colitis" describes an inflammation that involves the distal portion of the colon, extending as far as the splenic flexure. Sparing of the rectum or involvement of the right side (proximal portion) of the colon alone is unusual in UC. The inflammatory process of UC is limited to the colon and does not involve, for example, the small intestine, stomach, or esophagus. In addition, UC is distinguished by a superficial inflammation of the mucosa that generally spares the deeper layers of the bowel wall. Crypt abscesses, in which degenerated intestinal crypts are filled with neutrophils, are also typical of UC. A diagnosis of UC generally includes a colonoscopy to visualize and evaluate inflammation in the colon. A tissue biopsy may also be obtained during the procedure.

The variability of UC symptoms reflects differences in the extent of disease (*i.e.*, the amount of the colon and rectum that are inflamed) and the intensity of inflammation. The disease starts at the rectum and moves to the colon to involve more of the organ. UC can be categorized or graded according to the amount of colon involved. Patients with inflammation confined to the rectum and a short segment of the colon adjacent to the rectum tend to have milder symptoms and a better prognosis than patients with more widespread inflammation of the colon.

Ulcerative proctitis is a clinical subtype of UC defined by inflammation that is limited to the rectum. Proctosigmoiditis is a clinical subtype of UC which affects the rectum and the sigmoid colon. Left-sided colitis is a clinical subtype of UC which affects the entire left side of the colon, from the rectum to the place where the colon bends near the spleen and begins to run across the upper abdomen (the splenic flexure). Pancolitis is a clinical subtype of UC which affects the entire colon. Fulminant colitis is a rare, but severe form of pancolitis. Patients with fulminant colitis are extremely ill with dehydration, severe abdominal pain, protracted diarrhea with bleeding, and even shock.

Once UC is diagnosed, treatments including antibiotics, anti-inflammatory medications and anti-TNFα drugs can be administered to the patient. If these fail, prednisone can be used for a short period of time, but long-term use can be associated with significant side-effects. In order to maintain control of the disease, immunomodulators such as anti-TNFα drugs are used on a long-term basis. Flare-ups (*i.e.*, episodes of acute worsening) of the disease can often be treated by increasing the dosage of medications or adding new medications.

Surgery such as colectomy may be indicated for patients who have life-threatening complications of UC, such as massive bleeding, perforation, or infection. Surgery can be necessary for those non-responders who have the chronic form of the disease, and who fail to improve with medical therapy. In addition, patients who have long-standing ulcerative colitis may be candidates for removal of the large bowel, because of increased risk of developing cancer. More often, these patients are followed carefully with repeated colonoscopy and biopsy, and surgery is recommended when precancerous signs are identified.

It would be useful to have methods for determining whether a UC patient will respond to drug therapy or will need surgery. There is also a need for improved methods for prognosing clinical outcomes of UC and being able to predict whether surgery is required. The present invention satisfies these needs and provides related advantages as well.

Toedter et al., Am J. Gastroenterol. 2011; 106;1272-1280 discloses analyses of gene expression patterns of UC patients treated with infliximab.

**BRIEF SUMMARY OF THE INVENTION**

Disclosed herein is a method for predicting a clinical outcome of a subject having ulcerative colitis (UC). The method includes (a) determining a prognostic marker profile by detecting the presence or level of at least one prognostic marker selected from the group consisting of a cytokine, mucosal addressin cell adhesion molecule (MAdCAM-1), an anti-TNFα drug and a combination thereof in a sample from the subject; and (b) classifying the subject having UC as either a responder or a non-responder to an anti-TNFα drug therapy. The sample may be selected from the group consisting of whole blood, plasma, serum, urine, saliva, and another bodily fluid.

In some embodiments, the subject is classified as a non-responder if the level of MAdCAM-1 is higher or lower than a corresponding UC average MAdCAM-1 level.

In some embodiments, the cytokine is selected from the group consisting of tumor necrosis factor alpha (TNFα), interferon gamma (IFNγ), interleukin 1 beta (IL-1β), IL-2, IL-4, IL-6, IL-8, IL-10, IL-12p40, IL-12p70, IL-13 and a combination thereof.

In some embodiments, determining the prognostic marker profile further comprises calculating an IL-4 index value based on a sum (*i.e.*, algebraic sum) of the level of IFNγ and the level of IL-4 in the sample and/or calculating an IL-6 index value based on a sum of the level of IL-6 and the level of IL-8 in the sample. The subject can be classified as a non-responder if the IL-4 index value is higher compared to a corresponding UC average IL-4 index value. The subject can be classified as a responder if the IL-4 index value is equal or lower compared to the corresponding UC average IL-4 index value.

In some instances, determining the prognostic marker profile includes calculating an IL-4 ratio by dividing the IL-4 index value by the IL-6 index value. The subject can be classified as a non-responder if the IL-4 ratio is higher than a corresponding UC average IL-4 ratio. In some embodiments, the method also includes calculating an IL-4 mag ratio by multiplying the IL-4 index value and the IL-4 ratio. The subject can classified as a non-responder if the IL-4 mag ratio is higher than a corresponding UC average IL-4 mag ratio. In some instances, the subject is classified as a responder if the IL-4 mag ratio is equal to or lower than a corresponding UC average IL-4 mag ratio.

In other embodiments, determining the prognostic marker profile includes calculating an IFNγ index value based on a sum (*i.e.*, algebraic sum) of the level of IFNγ, the level of IL-4 and the level of IL-12p40 in the sample. The subject can be classified as a non-responder if the IFNγ index value is higher compared to a corresponding UC average IFNγ index value. Alternatively, the subject can be classified as a responder if the IFNγ index value is equal or lower compared to a corresponding UC average IFNγ index value.

In yet other embodiments, determining the prognostic marker profile includes calculating an IFNγ ratio by dividing the IFNγ index value by the IL-6 index value. The subject can be classified as a non-responder if the IFNγ ratio is higher than a corresponding UC average IFNγ ratio. In some instances, the method can include calculating an IFNγ mag ratio by multiplying the IFNγ index value and the IL-4 ratio. The subject is classified as a non-responder if the IFNγ mag ratio is higher than a corresponding UC average IFNγ mag ratio.

In some embodiments, determining the prognostic marker profile further comprises calculating a TNFα index value based on a sum of the level of TNFα, the level of IFNγ, the level of IL-4 and the level of IL-12p40 in the sample. The subject can be classified as a non-responder if the TNFα index value is higher compared to a corresponding UC average TNFα index value. Alternatively, the subject can be classified as a responder if the TNFα index value is equal or lower compared to the corresponding UC average TNFα index value.

In some embodiments, the non-responding subject (*i.e.*, the subject classified as a non-responder according to the method described herein) is likely to require a colectomy.

In some embodiments, the subject has been administered the anti-TNFa drug therapy. In other embodiments, the subject has not been administered the anti-TNFa drug therapy. In some instances, the anti-TNFa drug therapy is selected from the group consisting of infliximab (REMICADE™), entanercept (ENBREL™), adalimumab (HUMIRA™), golimumab (SIMPONI®), certolizumab pergol (CIMZIA®), and a combination thereof.

In some embodiments, the prognostic marker profile is transformed into a corresponding or substantially equivalent score or value of an endoscopic scoring system.

Also, disclosed herein is a method of treating ulcerative colitis in a subject in need thereof. The method includes performing a colectomy on a subject having ulcerative colitis and an IL-4 index value, an IFNγ index value and/or a TNFα index value higher than a corresponding UC average index value. In some embodiments, the IL-4 index value is a sum (*i.e.*, algebraic sum) of the level of IFNγ and the level of IL-4 in a sample from the subject. In some instances, the TNFα index value is a sum (*i.e.*, algebraic sum) of the level of TNFα, the level of IFNγ, the level of IL-4, and the level of IL-12p40 in a sample from the subject.

Additionally, disclosed herein is a method of treating ulcerative colitis in a subject in need thereof. The method includes performing a colectomy on a subject having ulcerative colitis and an IL-4 ratio, an IL-4 mag ratio, an IFNγ ratio and/or an IFNγ mag ratio higher than a corresponding UC average IL-4 ratio, IL-4 mag ratio, IFNγ ratio, IFNγ mag ratio, respectively. In some embodiments, the IL-4 ratio corresponds to an IL-4 index value divided by an IL-6 index value, wherein the IL-4 index value is a sum (*i.e.*, algebraic sum) of the level of IFNγ and the level of IL-4 in a sample from the subject, and the IL-6 index value is a sum of the level of IL-6 and the level of IL-8 in the sample. An IL-4 mag ratio can be the IL-4 ratio multiplied by the IL-6 index.

In some embodiments, the IFNγ ratio corresponds to an IFNγ index value divided by an IL-6 index value, wherein the IFNγ index value is a sum (*i.e.*, algebraic sum) of the level of IFNγ, the level of IL-4 and the level of IL-12p40 in a sample from the subject, and the IL-6 index value is a sum (*i.e.*, algebraic sum) of the level of IL-6 and the level of IL-8 in the sample. An IFNγ mag ratio can be the IFNγ ratio multiplied by the IL-6 index.

Also disclosed herein is a method for treating ulcerative colitis in a subject in need thereof. The method includes performing a colectomy on a subject having ulcerative colitis and a level of MAdCAM-1 that is higher or lower than a corresponding UC average MAdCAM-1 level.

Other objects, features, and advantages of the present invention will be apparent to one of skill in the art from the following detailed description and figures.

**BRIEF DESCRIPTION OF THE DRAWINGS**

FIG. 1 illustrates a cluster analysis of the data in presented in Table 5.

FIG. 2 shows a correlation between IFN$\gamma$ at baseline and after colectomy.

FIG. 3 shows that elevated TNF$\alpha$ levels at 24 hours after initial TNF$\alpha$ treatment are associated with receiving a colectomy.

FIG. 4 shows that elevated TNF$\alpha$ levels at 1 week after initial TNF$\alpha$ treatment are associated with receiving a colectomy.

FIG. 5 shows that changes of TNF$\alpha$ levels over time can be stratified according to whether the subject received or did not receive a colectomy.

FIG. 6 illustrates baseline levels of TNF$\alpha$ in patients who received colectomy (Y) and patients who did not (N).

FIG. 7 illustrates levels of TNF$\alpha$ at 24 hours after initial TNF$\alpha$ infusion in patients who received colectomy (Y) and patients who did not (N).

**DETAILED DESCRIPTION OF THE INVENTION**

The present invention provides methods for predicting a clinical outcome of a subject diagnosed as having ulcerative colitis (UC) or afflicted with UC. For instance, the method can be used to predict the likelihood that a subject will respond or will not respond to an anti-TNF$\alpha$ drug therapy. If it is determined that a subject will not respond to an anti-TNF$\alpha$ drug therapy, an alternative treatment such as surgery, *e.g.*, colectomy can be performed on the subject to treat UC.

In some aspects, disclosed are methods for selecting a subject with UC to receive a colectomy to treat the disease. Also disclosed are methods for selecting a subject with UC to be administered an anti-TNF$\alpha$ drug therapy. The present disclosure describes a method for treating UC by performing a colectomy on a UC subject with a designated prognostic marker profile as described herein. In certain aspects, the disclosure also provides a method of associating or transforming a prognostic profile to a colonoscopy disease activity assessment scale or index value.

**I. Definitions**

As used herein, the following terms have the meanings ascribed to them unless specified otherwise.

The term "colectomy" refers to surgical resection of any extent of the large intestine (colon). As used herein, the term includes, but is not limited to, right hemicolectomy, left hemicolectomy, extended hemicolectomy, transverse colectomy, sigmoidectomy, proctosigmoidectomy, Hartmann operation, "double-barrel" or Mikulicz colostomy, total colectomy, total proctocolectomy and subtotal colectomy.

The term "mucosal healing" includes generally the absence of visible ulcers on endoscopy, or endoscopic remission without blood, ulcers, erosions, or friability in each segment examined on endoscopy. The term also includes improved endoscopic features, particularly in previously inflamed areas; normal mucosa with pseudopolyps; and histological healing. In trials of therapeutic agents, mucosal healing has been defined as a Mayo Endoscopic Subscore of 0 or 1 after therapy, in patients who scored 2 or more before (See, Sandborn, Gastroenterology, 2014m 146:96-109).

The terms "anti-TNF$\alpha$ drug" or "TNF$\alpha$ inhibitor" as used herein is intended to encompass agents including proteins, antibodies, antibody fragments, fusion proteins (*e.g.*, lg fusion proteins or Fc fusion proteins), multivalent binding proteins (*e.g.*, DVD Ig), small molecule TNF$\alpha$ antagonists and similar naturally- or nonnaturally-occurring molecules, and/or recombinant and/or engineered forms thereof, that, directly or indirectly, inhibit TNF$\alpha$ activity, such as by inhibiting interaction of TNF$\alpha$ with a cell surface receptor for TNF$\alpha$, inhibiting TNF$\alpha$ protein production, inhibiting TNF$\alpha$ gene expression, inhibiting TNF$\alpha$ secretion from cells, inhibiting TNF$\alpha$ receptor signaling or any other means resulting in decreased TNF$\alpha$ activity in a subject. The term "anti-TNF$\alpha$ drug" or "TNF$\alpha$ inhibitor" preferably includes agents which interfere with TNF$\alpha$ activity. Examples of anti-TNFa drugs include, without limitation, infliximab (REMICADE™, Janssen Biotech), adalimumab (D2E7/HUMIRA™, Abbott Laboratories), etanercept (ENBREL™, Amgen), certolizumab pegol (CIMZIA®, UCB, Inc.), golimumab (SIMPONI®; CNTO 148), CDP 571 (Celltech), CDP 870 (Celltech), as well as other compounds which inhibit TNF$\alpha$ activity, such that when administered to a subject suffering from or at risk of suffering from a disorder in which TNF$\alpha$ activity is detrimental (*e.g.*, RA), the disorder is treated.

The term "responder" in the context of a treatment refers to an individual having a disease/condition who is responding to the administration of a specific treatment, *e.g.*, an anti-TNF$\alpha$ drug to treat the disease/condition. A responder to a specific treatment may experience a clinically relevant benefit from the treatment. In some instances, an individual who is responding to a drug treatment experiences any delay in onset of a disease/condition, reduction in the frequency or severity of adverse symptoms associated with a disease/condition, or amelioration, decrease or inhibition of a disease/condition or one or more symptoms associated with a disease/condition. A response to treatment can be compared to an individual not receiving a given treatment, or to the same patient prior to, or after cessation of, treatment.

The term "non-responder" in the context of a treatment refers to an individual having a disease/condition who is refractory to or intolerant to a specific therapy, *e.g.*, an anti-TNFα drug to treat the disease. A non-responder to a specific treatment may experience a little to no clinically relevant benefit from the treatment. In some instances, a non-responder does not experience a reduction in the frequency or severity of adverse symptoms associated with a disease/condition, or an amelioration, decrease or inhibition of a disease/condition or one or more symptoms associated with a disease/condition after receiving a given treatment.

The term "endoscopic scoring system" refers to a clinical evaluation system used to quantify the endoscopic features of ulcerative colitis in an individual. An endoscopic scoring system is useful for assessing disease activity, extent, behavior, progression and/or regression. Such a system can be used for UC diagnosis, prognosis, and assessing response to treatment.

The term "marker" includes any biochemical marker, protein marker, serological marker, genetic marker, or other clinical or echographic characteristic that can be used to classify a sample from an individual as a UC sample.

The term "cytokine" refers to a low-molecular weight polypeptide that is synthesized and secreted by specific T cells upon antigen recognition. A cytokine can regulate the nature, duration and intensity of an immune response by acting on lymphocytes and other cells. The term "cytokine" includes any of a variety of polypeptides or proteins secreted by immune cells that regulate a range of immune system functions and encompasses small cytokines such as chemokines. The term "cytokine" also includes adipocytokines, which comprise a group of cytokines secreted by adipocytes that function, for example, in the regulation of body weight, hematopoiesis, angiogenesis, wound healing, insulin resistance, the immune response, and the inflammatory response. Non-limiting examples of cytokines include IL-1, IL-1α, IL-1β, IL-2, IL-3, IL-4, IL-5, IL-6, IL-7, IL-8, IL-9, IL-10, IL-11, IL-12p40, IL-12p70, IL-13, IL-15, IL-16, IL-17, IL-18, IL-19, IL-20, IL-21, IL-22, IL-23, IFNα, IFNβ, IFNγ, TNFα, TNFβ, TGF-β1, M-CSF, G-CSF, GM-CSF, cKit, LIF CNTF, CD4, NGF, FAS, RANTES, MIP-1, PF4, MCAF, NAP2 and others described in, for example, Dinarello, Eur J Immunol, 2007, 37(1):S34-S45; Neurath, Nat Rev Immunol, 2014, 14:329-342 and Strober and Fuss, Gastroenterology, 2011, 140(6):1756-1767.

The term "TNFα" refers to a human tumor necrosis factor alpha cytokine that exists as a 17 kDa secreted form and a 26 kDa membrane associated form, the biologically active form of which is composed of a trimer of noncovalently bound 17 kDa molecules. The structure of TNFα is described further in, for example, Jones et al., Nature, 338:225-228 (1989). Human TNFα consists of a 35 amino acid (aa) cytoplasmic domain, a 21 aa transmembrane segment, and a 177 aa extracellular domain (ECD) (Pennica, D. et al. (1984) Nature 312:724). Within the ECD, human TNFα shares 97% aa sequence identity with rhesus TNFα, and 71% to 92% aa sequence identity with bovine, canine, cotton rat, equine, feline, mouse, porcine, and rat TNFα.

The term "mucosal addressin cell adhesion molecule-1" or "MAdCAM-1" refers to a cell-surface Ig superfamily member composed of two extracellular Ig domains, followed by a mucin-like domain, a transmembrane domain and a short cytoplasmatic domain. The MAdCAM-1 polypeptide interacts via its N-terminal Ig domain with the lymphocyte homing receptor integrin alpha4beta7. MAdCAM-1 promotes the adhesion of T and B cells, monocytes/macrophages, and potentially eosinophils, basophils, and differentiated mast cells to the vascular endothelium and is critical for lymphocyte homing to the gut. The MAdCAM-1 protein (60 kDa) is widely expressed on endothelia in both lymphoid and non-lymphoid tissues. In sera of healthy individuals, soluble MAdCAM-1 can be about 236.5 ± 55.8 ng/ml. In urine of healthy individuals, soluble MAdCAM-1 can be about 20-123 ng/ml. Measurement of soluble MAdCAM-1 levels is potentially useful to monitor disease activity and the results of therapy. An ELISA is available from Hycult biotech (Catalog #: HK337-02).

The term "prognostic marker profile" or "prognostic profile" includes the presence or level of 1, 2, 3, 4, 5, 6, 7, 8, 9, 10, 11, 12, 13, 14, 15, 16, 17, 18, 19, 20, 21, 22, 23, 24, 25, 30, 35, 40, 45, 50, or more marker(s) of an individual, wherein the marker(s) can be a serological marker, a protein marker, a genetic marker, and the like. The marker(s) can be predictive of an individual's disease prognosis, progression, regression and/or response to a particular therapy.

The term "prognosis" in the context of UC refers to a prediction of the probable course and outcome of UC or the likelihood of remission or recovery from the disease. For example, the prognosis can be surgery, development of a clinical subtype UC, development of one or more clinical factors, development of intestinal cancer, or recovery from the disease. In some embodiments, the methods are used to determine whether the individual will develop or progress to mild, mild to moderate, moderate, moderate to severe, severe or fulminant UC.

The term "diagnosis" in the context of UC refers to the determination that an individual suffers from UC. The term includes assessing the level of disease activity in an individual. In some cases, a diagnosis of UC includes determining whether the individual has mild, mild to moderate, moderate, moderate to severe, severe or fulminant UC.

The term "UC average" includes measuring the concentration or level of a "prognostic marker" in a cohort of patients having been diagnosed with UC and then taking the average value of the marker. The cohort of patients may contain 1, 2, 3, 4, 5, 6, 7, 8, 9, 10, 12, 15, 20, 25, 30, 40, 50 or more individual patients diagnosed with UC.

The term "classifying" includes "associating" or "categorizing" a sample or an individual with a disease state or prognosis. In certain instances, "classifying" is based on statistical evidence, empirical evidence, or both. In some instances, "classifying" is akin to diagnosing the disease state and/or differentiating the disease state from another disease state. In other instances, "classifying" is akin to providing a prognosis of the disease state in an individual diagnosed with the disease state.

The term "monitoring the progression or regression of UC" includes the use of the methods of the present invention to determine the disease state (*e.g.*, severity of UC) of an individual. In some aspects, the methods of the present invention can also be used to predict the progression of UC, *e.g.*, by determining a likelihood for UC to progress either rapidly or slowly in an individual based on the presence or level of at least one marker in a sample. In other aspects, the methods of the present invention can also be used to predict the regression of UC, *e.g.*, by determining a likelihood for UC to regress either rapidly or slowly in an individual based on the presence or level of at least one marker in a sample.

The term "monitoring drug efficacy in an individual receiving a drug useful for treating UC" includes the use of the methods of the present invention to determine the disease state (*e.g.*, severity of UC) of an individual after a therapeutic agent for treating UC has been administered, a drug useful for treating UC is any compound or drug used to improve the health of the individual and includes, without limitation, UC drugs such as aminosalicylates (*e.g.*, mesalazine, sulfasalazine, and the like), corticosteroids (*e.g.*, prednisone), thiopurines (*e.g.*, azathioprine, 6-mercaptopurine, and the like), methotrexate, monoclonal antibodies, anti-TNFα drugs (*e.g.*, infliximab), pharmaceutically acceptable salts thereof, derivatives thereof, analogs thereof, and combinations thereof.

The term "course of therapy" includes any therapeutic approach taken to relieve or prevent one or more symptoms associated with UC. The term encompasses administering any compound, drug, procedure, or regimen useful for improving the health of an individual with UC and includes any of the therapeutic agents as well as surgery. One skilled in the art will appreciate that either the course of therapy or the dose of the current course of therapy can be changed, *e.g.*, based upon the methods of the present invention.

The term "sample" refers to any biological specimen obtained from an individual. Suitable samples for use in the present invention include, without limitation, whole blood, plasma, serum, saliva, urine, stool, tears, any other bodily fluid, tissue samples (*e.g.*, biopsy), and cellular extracts thereof (*e.g.*, red blood cellular extract). In a preferred embodiment, the sample is a serum sample. The use of samples such as serum, saliva, and urine is well known in the art (*see, e.g.*, Hashida et al., J. Clin. Lab. Anal., 11:267-86 (1997)). One skilled in the art will appreciate that samples such as serum samples can be diluted prior to the analysis of marker levels.

The term "individual," "subject," or "patient" typically refers to humans, but also to other animals including, *e.g.*, other primates, rodents, canines, felines, equines, ovines, porcines, and the like.

## II. Detailed Description of Embodiments

### A. Detecting Prognostic Markers

In one aspect, the prognostic marker used to classify a sample from an individual includes a cytokine, mucosal addressin cell adhesion molecule (MAdCAM-1), an anti-TNFα drug, and any combination thereof. The method disclosed herein includes detecting the presence or level of a cytokine, and optionally, MAdCAM-1 or an anti-TNFa drug in a sample obtained from a subject. The presence or level of a cytokine and MAdCAM-1, or a cytokine and an anti-TNFα drug can be measured. Alternatively, the presence or level of MAdCAM-1 and/or an anti-TNFα drug can be determined. In some embodiments, the presence or level of a cytokine, MAdCAM-1, and an anti-TNFα drug is measured .

In some embodiments, one or more cytokines are detected. For example, the cytokine can be selected from TNFα, IFNγ, IL-1β, IL-2, IL-4, IL-6, IL-8, IL-10, IL-12p40, IL-12p70, IL-13, or any combination thereof. In some instances, the presence or level of 1, 2, 3, 4, 5, 6, 7, 8, 9, 10, 11 or more different cytokines are detected in a sample. The presence or level of TNFα, IFNγ, IL-4, IL-6, IL-8, IL-10, IL-12p40, IL-13, or any combination thereof can be measured. In some embodiments, the presence or level of TNFα, IFNγ, IL-4, IL-6, IL-8 and IL-12p40 are detected to classify a subject as being a responder or a non-responder to drug therapy. In other embodiments, the presence or level of IL-6 and IL-8 are detected. In yet other embodiments, the presence or level of IFNγ, IL-4 and IL-12p40 are detected. In some embodiments, the presence or level of TNFα, IFNγ, IL-4, and IL-12p40 are detected.

In certain aspects, the presence or level of at least one cytokine including, but not limited to, TNFα, TNF-related weak inducer of apoptosis (TWEAK), osteoprotegerin (OPG), IFN-α, IFN-β, IFNγ, IL-1α, IL-1β, IL-1 receptor antagonist (IL-1ra), IL-2, IL-4, IL-5, IL-6, soluble IL-6 receptor (sIL-6R), IL-7, IL-8, IL-9, IL-10, IL-12p40, IL-12p70, IL-13, IL-15, IL-17, IL-23, and IL-27 is determined in a sample. In certain other aspects, the presence or level of at least one chemokine such as, for example, CXCL1/GRO1/GROα, CXCL2/GRO2, CXCL3/GRO3, CXCL4/PF-4, CXCL5/ENA-78, CXCL6/GCP-2, CXCL7/NAP-2, CXCL9/MIG, CXCL10/IP-10, CXCL11/I-TAC, CXCL12/SDF-1, CXCL13/BCA-1, CXCL14/BRAK, CXCL15, CXCL16, CXCL17/DMC, CCL1, CCL2/MCP-1, CCL3/MIP-1α, CCL4/MIP-1β, CCL5/RANTES, CCL6/C10, CCL7/MCP-3, CCL8/MCP-2, CCL9/CCL10, CCL11/Eotaxin, CCL12/MCP-5, CCL13/MCP-4, CCL14/HCC-1, CCL15/MIP-5, CCL16/LEC, CCL17/TARC, CCL18/MIP-4, CCL19/MIP-3β, CCL20/MIP-3α, CCL21/SLC, CCL22/MDC, CCL23/MPIF1, CCL24/Eotaxin-2, CCL25/TECK, CCL26/Eotaxin-3, CCL27/CTACK, CCL28/MEC, CL1, CL2, and $CX_3CL1$ is determined in a sample. In certain further aspects, the presence or level of at least one adipocytokine including, but not limited to, leptin, adiponectin, resistin, active or total plasminogen activator inhibitor-1 (PAI-1), visfatin, and retinol binding protein 4 (RBP4) is determined in a sample.

In some embodiments, the level(s) of one or more cytokines, MAdCAM-1 and/or an anti-TNFα drug is detected before the subject is administered an anti-TNFα drug (baseline). In some cases, the levels are measured upon diagnosis of UC. In other cases, the levels are measured before diagnosis. Alternatively, the level(s) can be measured after the subject is administered an anti-TNFα drug. In some embodiments, the level(s) are measured 24 hours, 48 hours, 72 hours, 1 week, or 2 weeks after administration of the anti-TNFα drug. In other embodiments, the levels are measured more than 2 weeks after administration of drug.

In certain instances, commercial kits are available to measure cytokine levels and concentration. The instructions of the manufacturers are followed. All these tests are enzyme immunoassays. The principle is that microtiter plate wells are labeled with a cytokine specific capture antibody. If cytokine is present in the samples added to the wells it will be captured to the bottom of the well. To quantify how much cytokine that has been captured a second antibody, labeled with an enzyme may be added to the wells. The reaction may thereafter be measured as color developed after addition of a substrate for the enzyme. The values for the test wells may be compared with a standard curves obtained from a series of known amounts of cytokine. A dose response curve may be established for each substance.

In certain instances, the presence or level of a cytokine and/or MAdCAM-1 is determined using an immunoassay or an immunohistochemical assay. A non-limiting example of an immunoassay suitable for use in the method of the present invention includes an enzyme-linked immunosorbent assay (ELISA). Examples of immunohistochemical assays suitable for use in the method of the present invention include, but are not limited to, immunofluorescence assays such as direct fluorescent antibody assays, immunofluorescence antibody assays, anticomplement immunofluorescence assays, and avidin-biotin immunofluorescence assays. Other types of immunohistochemical assays include immunoperoxidase assays.

Suitable ELISA kits for determining the presence or level of a cytokine in a sample such as a serum, plasma, saliva, or urine sample are available from, e.g., R&D Systems®, Inc. (Minneapolis, MN), Neogen® Corp. (Lexington, KY), Alpco Diagnostics® (Salem, NH), Assay Designs® Inc. (Ann Arbor, MI), BD Pharmingen™ (San Diego, CA), Thermo Fisher Scientific® (Camarillo, CA), Calbiochem (San Diego, CA), CHEMICON International™ Inc. (Temecula, CA), Antigenix America™ Inc. (Huntington Station, NY), QIAGEN® Inc. (Valencia, CA), Bio-Rad® Laboratories Inc. (Hercules, CA), and/or Bender MedSystems® Inc. (Burlingame, CA). The following commercial kits may be used: IL-1β, IL-2, IL-4, IL-6, IL-8, IL-10, IFNγ, TNFα (BD OptEIA™; BD Pharmingen™), and soluble CD8 (T Cell Diagnostics®).

MAdCAM-1 levels can be measured by any method recognized by those skilled in the art. Non-limiting examples of useful methods include an immunoassay, e.g., ELISA, flow cytometry, and mass spectrometry. A commercially available ELISA kit for detecting MAdCAM-1 is available from Hycult® Biotech (Plymouth Meeting, PA) and BD Pharmingen™ (San Diego, CA).

Methods for detecting the presence or level of an anti-TNFα drug include an immunoassay, e.g., ELISA and fluid-phase radioimmunoassay, size exclusion chromatography, mass spectrometry, mobility shift assay, and those described in, e.g., U.S. Patent No. 8,574,855, and U.S. Patent Publication Nos. 2014/0051184 and 2013/029685 which disclose methods for measuring anti-TNFα drug levels. For example, size exclusion chromatography wherein molecules in solution are separated based on their size and/or hydrodynamic volume is useful for these measurements.

## B. Calculating Prognostic Marker Profiles

The level of one or more cytokines described herein can be weighted and in some instances, combined. In some embodiments, a prognostic marker value can be provided by weighting the determined level of each cytokine with a predefined coefficient, and the weighted levels can be combined to provide a prognostic marker value. The combining step can be either by straight addition or averaging (i.e., weighted equally) or by a different predefined coefficient.

The levels of one or more cytokines can be transformed or converted into a prognostic marker profile that can be used in the methods described herein. The prognostic marker profile can include an IL-4 index value, an IL-6 index value, an IL-4 ratio, an IL-4 mag ratio, an IFNγ index value, an IFNγ ratio, an IFNγ mag ratio, a TNFα index value or any combination thereof.

In some embodiments, an IL-4 index value is determined by adding the level of IFNγ and the level of IL-4. An IL-6 index value is determined by adding the level of IL-6 and the level of IL-8. An IL-4 ratio can be a quotient of the IL-4 index value and the IL-6 index value. An IL-4 mag ratio can be determined by multiplying the IL-4 index value and the IL-4 ratio.

In some embodiments, an IFNγ index value is determined by adding together the level of IFNγ, the level of IL-4 and the level of IL-12p40. In some instances, an IFNγ ratio is determined by dividing the IFNγ index value by the IL-6 index value. The IFNγ mag ratio is determined by multiplying the IFNγ index value and the IL-4 ratio.

In some embodiments, a TNFα index value is determined by adding together the level of TNFα, the level of IFNγ, the level of IL-4 and the level of IL-12p40.

Once generated, the test index value, ratio or mag ratio can be compared to one or more reference or threshold value(s), ratio(s) or mag ratio(s). respectively. In order to establish a reference value for practicing the method provided herein, a population of subject having UC can be used. In some embodiments, a population of UC patients receiving treatment for UC, *e.g.*, an anti-TNFα therapy can be used. Such patients may be responding to the therapy. In other embodiments, a population of UC patients receiving an anti-TNFα therapy and not requiring surgery, *e.g.*, colectomy is used. In some instances, the UC patients have a specific subtype of UC. In other instances, the UC patients have any subtype of UC. The patients may be within appropriate parameters, if applicable, for the purpose of predicting clinical outcome, selecting therapy, and/or predicting response to anti-TNFα therapy using the methods of the present disclosure. Optionally, the patients are of similar age or similar ethnic background. The status of the selected patients can be confirmed by well established, routinely employed methods including but not limited to general physical examination of the individuals and general review of their medical history.

The selected group of patients will generally be of sufficient size, such that the average value in the sample obtained from the group can be reasonably regarded as representative of a particular indication, for example, indicative of clinical response or not after a set period of time (*e.g.*, five years) after treatment.

Once an average value is established based on the individual values found in each subject of the selected group, this average or median or representative value or profile can be used as a reference value. In some embodiments, a reference value is determined using one or more statistical method described herein. A sample value over the reference value can indicate a more than average likelihood of lack of clinical response (non-response) to anti-TNFα therapy, whereas a sample value at or below the reference value can indicate an average or below-average likelihood of non-response to anti-TNFα therapy. In some embodiments, a standard deviation is also determined during the same process. In some cases, separate reference values may be established for separately defined groups having distinct characteristics such as age, gender, or ethnic background.

According to the methods described herein, the sample is compared to one or more reference or threshold values, *e.g.*, average reference index value, average ratio and average mag ratio. In some embodiments, the sample value is deemed "high" or "higher" if it is at least 1, 2, 3, 4, 5, 10, 15, 20 or more standard deviations greater than the reference value subjects. In other embodiments, the sample value is deemed "low" or "lower" if it is at least 1, 2, 3, 4, 5, 10, 15, 20 or more standard deviations lower than the reference or threshold value.

In some embodiments, a computer-based analysis program is used to translate the raw data generated by the detection methods described herein (*e.g.*, the presence, absence, or level of a given marker or markers) into a profile or score of predictive value to a clinician.

The score or profile, as determined according to the methods above, can predict that the patient has an above-average or high likelihood of response to an anti-TNFa therapy. In other embodiments, the score can predict that the patient has a below-average or low likelihood of response to an anti-TNFa therapy. Such a patient may require surgery, *e.g.*, colectomy to treat UC.

**C. Responsive to Anti-TNFa Therapies**

Disclosed herein is a method for predicting a positive clinical outcome such as clinical remission, endoscopic improvement, or low (*i.e.*, below average) risk for requiring colectomy in a patient with UC. In some embodiments, the patient is receiving an anti-TNFα drug. In other embodiments, the patient has not received an anti-TNFα drug. In some aspects, the method is used to classify UC patient as being a responder to an anti-TNFα drug. In other aspects, the method disclosed herein is used to determine if a UC patient administered an anti-TNFα drug therapy is responding or has a likelihood of responding to the therapy.

The method can be used to determine if the patient will clinically benefit from administration of a therapy comprising an anti-TNFα drug. In addition, provided herein is a method for selecting a treatment for a patient with UC. In some instances, the treatment comprises an anti-TNFα drug therapy or in combination of another therapy for UC.

In some embodiments, a subject is classified or determined to be a responder to an anti-TNFα drug therapy. In some instances, if a subject's IL-4 index value is equal to or lower than a reference IL-4 index value (*i.e.*, UC average IL-4 index value), the subject is classified as a responder. Also, if the subject's IL-4 ratio or IL-4 mag ratio is equal to or lower than a reference IL-4 ratio or a reference IL-4 mag ratio, respectively, the subject is considered a responder. In other embodiments, if the subject's IFNγ index value is equal to or lower than a reference IFNγ index value (*i.e.*, UC average IFNγ index value), the subject is classified as a responder. Similarly, an IFNγ ratio or IFNγ mag ratio that is equal to or lower than a reference IL-4 ratio or a reference IL-4 mag ratio, respectively indicates that the subject is a responder. In yet other embodiments, if the subject's TNFα index value is equal to or lower than a reference TNFα index value (*i.e.*, UC average TNFα index value), the subject is classified as a responder.

According to the methods disclosed herein, if a subject is a responder, an anti-TNFα drug therapy is administered to treat UC. Examples of anti-TNFα drug therapies include, without limitation, infliximab (REMICADE™, Janssen Biotech), adalimumab (D2E7/HUMIRA™, Abbott Laboratories), etanercept (ENBREL™, Amgen), certolizumab pegol (CIMZIA®, UCB, Inc.), golimumab (SIMPONI®; CNTO 148), and the like.

### D. Non-Responsive to Anti-TNFa Therapies

Disclosed herein a method for predicting a poor clinical outcome such as lack of clinical remission, lack of endoscopic improvement, or a high risk (*i.e.*, above average) for requiring colectomy in a patient with UC. In some embodiments, the patient is receiving an anti-TNFα drug. In other embodiments, the patient has not received an anti-TNFα drug.

In some aspects, the method is used to classify a patient as being a non-responder to an anti-TNFα drug. Optionally, the method includes classifying the patient as requiring surgery, *e.g.*, colectomy. In other aspects, the method disclosed herein is used to determine if a UC patient administered an anti-TNFα drug therapy is not responding or does not have a likelihood of responding to the drug. The methods are useful for identifying those patients who are likely to fail a treatment regimen comprising an anti-TNFα drug. The method can be used to identify patients with UC who will clinically benefit from surgery such as colectomy.

In one aspect, disclosed herein is a method for selecting a treatment for a patient with UC. In some instances, the treatment comprises colectomy. In some embodiments, the patient is receiving a treatment comprising an anti-TNFα drug.

In some embodiments, a subject is classified as a non-responder to an anti-TNFα drug therapy if the level of MAdCAM-1 is determined to be higher than a MAdCAM-1 reference value (*i.e.*, a UC average MAdCAM-1 value). In other embodiments, a subject is classified a non-responder is the subject's MAdCAM-1 level is lower than a MAdCAM-1 reference value. In some embodiments, if the subject's levels of TNFα, IFNγ, IL-4, IL-12p40, and any combination thereof are higher than corresponding reference levels, the subject is classified as a non-responder. For instance, if the levels of TNFα, IFNγ, IL-4 and IL-12p40; TNFα, IFNγ, and IL-4; TNFα, IFNγ, and IL-12p40; TNFα, IL-4, and IL-12p40; IFNγ, IL-4, and IL-12p40; TNFα and IFNγ; TNFα and IL-4; TNFα and IL-12p40; IFNγ and IL-4; IFNγ and IL-12p40; IL-4 and IL-12p40; TNFα; IFNγ; IL-4; and IL-12p40 are higher than the corresponding level(s), the subject is classified as a non-responder.

In some embodiments, if the level of the IL-4 index value is higher than a reference IL-4 index value, the subject is considered to be a non-responder. In other embodiments, a non-responder includes a subject having an IL-4 ratio or an IL-4 mag ratio that is higher than a reference IL-4 ratio or a reference IL-4 mag ratio. In yet other embodiments, a subject is a non-responder if the subject's IFNγ index value is higher than a reference IFNγ index value. In some instances, a higher IFNγ ratio or higher IFNγ mag ratio compared to a reference IFNγ ratio or a reference IFNγ mag ratio indicates that the subject is a non-responder to anti-TNFα drug therapy. In another embodiment, a non-responder has a TNFα index value that is higher than a reference TNFα index value.

According to the methods disclosed herein, a subject classified as a non-responder is likely to require a colectomy. In some embodiments, a non-responder is treated by undergoing a colectomy. If a subject with UC is classified as a non-responder to an anti-TNFα drug therapy, surgery, *e.g.*, colectomy can be performed on the subject to treat UC. Non-limiting examples of colectomy include total colectomy, partial colectomy, hemicolectomy and protocolectomy.

### E. Endoscopic Disease Activity Assessment Index

In certain aspects, the present disclosure provides a method of transforming or associating a prognostic profile to a colonoscopy disease activity assessment scale or index. Non-limiting examples of a colonoscopy disease activity scale are described in, for example, Paine, Gastroenterol i, 2014, 2(2): 161-168. The scale or index can be the Ulcerative Colitis Endoscopic Index of Severity (UCEIS) score, the Baron Score, the Ulcerative Colitis Colonoscopic Index of Severity (UCCIS), the Rachmilewitz Endoscopic Index, the Sutherland Index, the Matts Score, the Blackstone Index, the Mayo Index or a combination thereof.

For example, the Ulcerative Colitis Endoscopic Index of Severity (UCEIS) is an endoscopic scoring system that includes an assessment of vascular pattern, and bleeding. In this system, the vascular pattern is rated as 1-3 with 1 as normal; 2 as patchy loss of vascular pattern; and 3 as complete loss of vascular pattern. In addition, bleeding is characterized from 1-4 with 1 as no bleeding, mucosal bleeding as 2, mild colonic luminal bleeding as 3, and moderate or severe luminal bleeding as 4. Erosions and ulcers are characterized from 1-4 with 1 as no erosion and/or ulcers, 2 as erosions, 3 as superficial ulcerations, and 4 as deep ulcers (see, *e.g.*, Travis et al., Gut, 2012;61:535-42).

Another endoscopic scoring system is known as the Ulcerative Colitis Colonoscopic Index of Severity (UCCIS).

In this scoring system, vascular pattern, ulcerations, bleeding-friability, and granularity were all found to be good-to-excellent in predicting overall endoscopic severity and these components were used to make the UCCIS. Patients with a normal vascular pattern were given a score of 0, while those with a partial loss of pattern were given a 1, and patients with complete obliteration of vascular pattern were given a 2. Ulcerations were graded as absent (0), erosions or pinpoint ulcers (1), multiple shallow ulcers with mucus (2), deep ulcers (3), or diffuse ulcers involving more than 30% of the mucosa (4). In terms of bleeding and friability, mucosa with no bleeding or friability was designated 0, while mucosa with friability and bleeding with minimal touch was rated 1, and tissue with spontaneous bleeding was given 2. Granularity was divided into 0-3, with 0 corresponding with no granularity, 1 with fine granularity, and 2 with coarse granularity (see, *e.g.*, Thia KT, Inflamm Bowel Dis, 2011, 17:1257-64).

Yet another endoscopic scale is the Mayo score. The Mayo Score is a combined endoscopic scale and clinical scale used to assess the severity of UC. The Mayo Score is a composite of subscores from four categories, including stool frequency, rectal bleeding, findings of flexible proctosigmoidoscopy or colonoscopy, and physician's global assessment, with a total score ranging from 0-12. Within the endoscopic component of the Mayo Score, a score of 0 is given for normal mucosa or inactive UC, while a score of 1 is given for mild disease with evidence of mild friability, reduced vascular pattern, and mucosal erythema. A score of 2 is indicative of moderate disease with friability, erosions, complete loss of vascular pattern, and significant erythema, and a score of 3 indicates ulceration and spontaneous bleeding. Mucosal healing has been defined as a Mayo endoscopic subscore of 0 or 1 in major trials of biological therapies in UC. (see, *e.g.*, Schroeder, N Engl J Med, 1987, 317:1625-29).

In some embodiments, the prognostic marker profile such as an IL-4 index value, IL-4 ratio, IL-4 mag ratio, IFN$\gamma$ index value, IFN$\gamma$ ratio, IFN$\gamma$ mag ratio, and/or TNF$\alpha$ index value determined according to the methods described herein corresponds to a score or value on a clinical index such as a colonoscopy disease activity assessment scale or index. In some instances, a profile which indicates that the subject is a non-responder may correspond to a colonoscopy disease activity assessment score that also indicates that the subject is in need of a colectomy. A high prognostic marker profile may be associated with a high colonoscopy disease activity assessment score. For example, a high IL-4 mag ratio or a high IFN$\gamma$ mag ratio can be transformed by algorithmic or computational methods to a high UCCIS score, such as a numerical value of 24-34.

In some embodiments, a prognostic marker profile can predict the presence or absence of mucosal healing. For instance, a prognostic marker profile that indicates that the subject is a responder to an anti-TNFa therapy can also indicate that the subject is undergoing mucosal healing. Alternatively, a prognostic marker profile that indicates that the subject is a non-responder can indicated that the subject is not undergoing or experiencing mucosal healing.

## F. Statistical Methods

In some aspects, one or more statistical algorithms such as, *e.g*, learning statistical classifier systems are applied to the presence, absence, and/or level of one or more prognostic markers determined by any of the assays described herein to determine response or non-response. In some aspects, one or more statistical algorithms are applied to the presence, absence, or level of one or more markers to predict a clinical outcome from a sample of a subject having ulcerative colitis (UC). In certain aspects, the determination is whether the individual is a responder or non-responder. In some embodiments, the statistical algorithm(s) described herein can be used to determine the prognostic profile of a test subject and/or one or more reference subjects.

The term "statistical analysis" or "statistical algorithm" or "statistical process" includes any of a variety of statistical methods and models used to determine relationships between variables. In the present invention, the variables are the presence, absence, or level of at least one marker (*e.g.*, serological or clinical) of interest. Any number of markers can be analyzed using a statistical analysis described herein. For example, the presence, absence, or level of 1, 2, 3, 4, 5, 6, 7, 8, 9, 10, 11, 12, 13, 14, 15, 16, 17, 18, 19, 20, 25, 3 0, 3 5, 40, 45, 50, 55, 60, or more markers can be included in a statistical analysis. In one embodiment, logistic regression is used. In another embodiment, linear regression is used.

In certain embodiments, the statistical analyses of the present invention comprise a quantile measurement of one or more markers, *e.g.*, within a given population, as a variable. Quantiles are a set of "cut points" that divide a sample of data into groups containing (as far as possible) equal numbers of observations. For example, quartiles are values that divide a sample of data into four groups containing (as far as possible) equal numbers of observations. The lower quartile is the data value a quarter of the way up through the ordered data set; the upper quartile is the data value a quarter of the way down through the ordered data set. Quintiles are values that divide a sample of data into five groups containing (as far as possible) equal numbers of observations. The present invention can also include the use of percentile ranges of marker levels (*e.g.*, tertiles, quartile, quintiles, *etc.*), or their cumulative indices (*e.g.*, quartile sums of marker levels to obtain quartile sum scores (QSS), *etc.*) as variables in the statistical analyses (just as with continuous variables).

In particular embodiments, the statistical analysis comprises one or more learning statistical classifier systems. As used herein, the term "learning statistical classifier system" includes a machine learning algorithmic technique capable of adapting to complex data sets (*e.g.*, panel of markers of interest) and making decisions based upon such data sets.

In some embodiments, a single learning statistical classifier system such as a decision/classification tree (*e.g.*, random forest (RF) or classification and regression tree (CART)) is used. In other embodiments, a combination of 2, 3, 4, 5, 6, 7, 8, 9, 10, or more learning statistical classifier systems are used, preferably in tandem. Examples of learning statistical classifier systems include, but are not limited to, those using inductive learning (*e.g.*, decision/classification trees such as random forests, classification and regression trees (CART), boosted trees, *etc.*), Probably Approximately Correct (PAC) learning, connectionist learning (*e.g.*, neural networks (NN), artificial neural networks (ANN), neuro fuzzy networks (NFN), network structures, perceptrons such as multi-layer perceptrons, multi-layer feed-forward networks, applications of neural networks, Bayesian learning in belief networks, *etc.*), reinforcement learning (*e.g.*, passive learning in a known environment such as naive learning, adaptive dynamic learning, and temporal difference learning, passive learning in an unknown environment, active learning in an unknown environment, learning action-value functions, applications of reinforcement learning, *etc.*), and genetic algorithms and evolutionary programming. Other learning statistical classifier systems include support vector machines (*e.g.*, Kernel methods), multivariate adaptive regression splines (MARS), Levenberg-Marquardt algorithms, Gauss-Newton algorithms, mixtures of Gaussians, gradient descent algorithms, and learning vector quantization (LVQ).

Random forests are learning statistical classifier systems that are constructed using an algorithm developed by Leo Breiman and Adele Cutler. Random forests use a large number of individual decision trees and decide the class by choosing the mode (*i.e.*, most frequently occurring) of the classes as determined by the individual trees. Random forest analysis can be performed, *e.g.*, using the RandomForests software available from Salford Systems (San Diego, CA). *See, e.g.*, Breiman, Machine Learning, 45:5-32 (2001); and http://stat-www.berkeley.edu/users/breiman/Random-Forests/cc_home.htm, for a description of random forests.

Classification and regression trees represent a computer intensive alternative to fitting classical regression models and are typically used to determine the best possible model for a categorical or continuous response of interest based upon one or more predictors. Classification and regression tree analysis can be performed, *e.g.*, using the C&RT software available from Salford Systems or the Statistica data analysis software available from StatSoft, Inc. (Tulsa, OK). A description of classification and regression trees is found, *e.g.,* in Breiman et al. "Classification and Regression Trees," Chapman and Hall, New York (1984); and Steinberg et al., "CART: Tree-Structured Non-Parametric Data Analysis," Salford Systems, San Diego, (1995).

Neural networks are interconnected groups of artificial neurons that use a mathematical or computational model for information processing based on a connectionist approach to computation. Typically, neural networks are adaptive systems that change their structure based on external or internal information that flows through the network. Specific examples of neural networks include feed-forward neural networks such as perceptrons, single-layer perceptrons, multi-layer perceptrons, backpropagation networks, ADALINE networks, MADALINE networks, Learnmatrix networks, radial basis function (RBF) networks, and self-organizing maps or Kohonen self-organizing networks; recurrent neural networks such as simple recurrent networks and Hopfield networks; stochastic neural networks such as Boltzmann machines; modular neural networks such as committee of machines and associative neural networks; and other types of networks such as instantaneously trained neural networks, spiking neural networks, dynamic neural networks, and cascading neural networks. Neural network analysis can be performed, *e.g.*, using the Statistica data analysis software available from StatSoft, Inc. *See, e.g.*, Freeman et al., In "Neural Networks: Algorithms, Applications and Programming Techniques," Addison-Wesley Publishing Company (1991); Zadeh, Information and Control, 8:338-353 (1965); Zadeh, "IEEE Trans. on Systems, Man and Cybernetics," 3:28-44 (1973); Gersho et al., In "Vector Quantization and Signal Compression," Kluywer Academic Publishers, Boston, Dordrecht, London (1992); and Hassoun, "Fundamentals of Artificial Neural Networks," MIT Press, Cambridge, Massachusetts, London (1995), for a description of neural networks.

Support vector machines are a set of related supervised learning techniques used for classification and regression and are described, *e.g.*, in Cristianini et al., "An Introduction to Support Vector Machines and Other Kernel-Based Learning Methods," Cambridge University Press (2000). Support vector machine analysis can be performed, *e.g.*, using the SVM*light* software developed by Thorsten Joachims (Cornell University) or using the LIBSVM software developed by Chih-Chung Chang and Chih-Jen Lin (National Taiwan University).

The various statistical methods and models described herein can be trained and tested using a cohort of samples (*e.g.*, biological samples) from individuals that a clinical outcome from a sample of a subject having ulcerative colitis (UC) is known (responders) and patients that do not experience response (non-responders) within a given time interval. One skilled in the art will know of additional techniques and diagnostic criteria for obtaining a cohort of patient samples that can be used in training and testing the statistical methods and models of the present invention.

As used herein, the term "sensitivity" refers to the probability that a method of the present invention gives a positive result when the sample is positive. Sensitivity is calculated as the number of true positive results divided by the sum of the true positives and false negatives. In certain instances, sensitivity is a measure of how well the present invention correctly predicts those patients who will experience response or non-response. The statistical methods and models can be selected such that the sensitivity is at least about 60%, and can be, *e.g.*, at least about 65%, 70%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%.

The term "specificity" refers to the probability that a method of the invention gives a negative result when the sample is not positive. Specificity is calculated as the number of true negative results divided by the sum of the true negatives and false positives. In certain instances, specificity is a measure of how well of how well the present invention correctly predicts those patients who will experience response or non-response. The statistical methods and models can be selected such that the specificity is at least about 60%, and can be, e.g., at least about 65%, 70%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%.

The term "negative predictive value" or "NPV" refers to the probability that an individual predicted not to experience response actually does experience response. Negative predictive value can be calculated as the number of true negatives divided by the sum of the true negatives and false negatives.

As used herein, the term "positive predictive value" or "PPV" refers to the probability that an individual predicted to experience response actually experiences response. Positive predictive value can be calculated as the number of true positives divided by the sum of the true positives and false positives. Positive predictive value is determined by the characteristics of the method as well as the prevalence of the disease in the population analyzed. The statistical methods and models can be selected such that the positive predictive value in a population having a disease prevalence is in the range of about 70% to about 99% and can be, for example, at least about 70%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%.

Predictive values, including negative and positive predictive values, are influenced by the frequency of recurrence in the population analyzed. In the present invention, the statistical methods and models can be selected to produce a desired clinical parameter for a clinical population with a particular frequency of response.

### III. Examples

The following examples are offered to illustrate, but not to limit, the claimed invention.

### Example 1 shows Baseline Data.

Table 1 below shows the Baseline Diagnostic Data for 7 individuals. The raw data is from 7 individuals having previously been diagnosed with UC. The baseline value for each diagnostic marker is before therapy began and after diagnosis. This example shows that certain diagnostic markers can be measured before initiation of therapy to determine whether the individual is a responder to anti-TNF$\alpha$ therapy or a non-responder to such therapy. Non-responders are likely to require surgery.

**Table 1. Baseline Raw Data**

| | Assay | 50 | 2,000,000 | 1110 | 400 | 170 | 604 | 398 | 254 | 2400 |
|---|---|---|---|---|---|---|---|---|---|---|
| | Limit | 0.3 | 1186 | 0.854 | 0.309 | 0.131 | 0.466 | 0.307 | 0.196 | 5.59 |
| ID | Timepoint | TNF$\alpha$ (pg/ml) | MAdCAM-1 (pg/ml) | IFN$\gamma$ (pg/mL) | IL-1$\beta$ (pg/mL) | IL-4 (pg/mL) | IL-6 (pg/mL) | IL-8 (pg/mL) | IL-10 (pg/mL) | IL-12p40 (pg/mL) |
| Patient 2 | Baseline | 7.91 | 195,936 | 18.371 | 0.719 | 0.476 | 1.727 | 11.535 | 24.257 | 11.79 |
| Patient 7 | Baseline | 5.58 | 40,756 | 20.731 | 0.008 | 0.198 | 0.424 | 6.123 | 0.077 | 29.08 |
| Patient 1 | Baseline | 7.16 | 53,178 | 2.119 | 0.455 | 0.086 | 4.080 | 38.546 | 2.832 | 8.65 |
| Patient 8 | Baseline | 2.85 | 118,116 | 10.150 | 0.199 | 0.060 | 4.598 | 24.145 | 0.391 | 131.23 |
| Patient 6 | Baseline | 2.02 | 105,392 | 21.067 | 0.098 | 0.000 | 5.626 | 11.542 | 1.716 | 27.97 |
| Patient 3 | Baseline | 8.58 | 112,060 | 3.460 | 0.037 | 0.000 | 0.978 | 8.735 | 2.955 | 4.39 |
| Patient 5 | Baseline | 1.97 | 124,868 | 3.579 | 0.100 | 0.246 | 0.546 | 5.615 | 1.500 | 9.81 |

In this example, the diagnostic markers measured at baseline were TNFα, MAdCAM-1, IFNγ, IL-1β, IL-4, IL-6, IL-8, IL-10, and IL-12p40.

Patients 2 and 7 required surgery.

Table 2 below shows the Baseline Normalized Diagnostic Data for the 7 individuals. Two (2) individuals (patient 2 and patient 7) required colectomies and 5 patients did not receive colectomies. The data has been normalized by dividing each diagnostic marker value by the maximum data value of that diagnostic marker.

Table 2. Baseline Normalized* Data for each Marker

| ID | Timepoint | TNFα | MAdCAM-1 | IFNγ | IL-β | IL-4 | IL-6 | IL-8 | IL-10 | IL-12p40 |
|---|---|---|---|---|---|---|---|---|---|---|
| Patient 2 | Baseline | 0.92 | 1.57 | 0.87 | 1.58 | 1.94 | 0.31 | 0.30 | 8.21 | 0.09 |
| Patient 7 | Baseline | 0.65 | 0.33 | 0.98 | 0.02 | 0.81 | 0.08 | 0.16 | 0.03 | 0.22 |
| Patient 1 | Baseline | 0.83 | 0.43 | 0.10 | 1.00 | 0.35 | 0.73 | 1.00 | 0.96 | 0.07 |
| Patient 8 | Baseline | 0.33 | 0.95 | 0.48 | 0.44 | 0.25 | 0.82 | 0.63 | 0.13 | 1.00 |
| Patient 6 | Baseline | 0.23 | 0.84 | 1.00 | 0.21 | 0.00 | 1.00 | 0.30 | 0.58 | 0.21 |
| Patient 3 | Baseline | 1.00 | 0.90 | 0.16 | 0.08 | 0.00 | 0.17 | 0.23 | 1.00 | 0.03 |
| Patient 5 | Baseline | 0.23 | 1.00 | 0.17 | 0.22 | 1.00 | 0.10 | 0.15 | 0.51 | 0.07 |
| *=value/max | | | | | | | | | | |

Example 1 shows that the values of MAdCAM-1 for the cohort requiring surgery are either extremely high, or extremely low compared to the cohort that responded to anti-TNFα therapy. As such, MAdCAM-1 is a marker of characteristic value in determining an individual's ability to respond to therapy.

Example 1 further shows that the normalized values for MAdCAM-1 for the responding cohort were between 0.43 and 1.0.

**Example 2 shows the use of IL-4 and IL-6 Indexes.**

Table 3 below shows the calculation of an IL-4 index and an IL-6 index. The cohort of patients with a high IL-4 index compared to the UC average required surgery. Further, patients with a high ratio or mag ratio compared to the UC average required surgery.

The IL-4 index is defined as the sum of the IFNγ level value and the IL-4 level value.

The IL-6 index is defined as the sum of the IL-6 level value and the IL-8 level value.

Table 3 also includes the "IL-4 ratio" in column 6, which is the quotient of the IL-4 index and the IL-6 index (*e.g.*, IL-4 index/the IL-6 index).

Table 3 also includes the "IL-4 mag ratio" in column 7, which is the product of the IL-4 index multiplied by the IL-4 ratio.

Table 3. IL-4 Index Value, Ratio and Mag Ratio Calculations

| Subject ID | Time point | Colectomy | IFN-γ+ IL-4 (IL-4 index) | IL-6 + IL-8 (IL-6 index) | IL-4 Ratio* | IL-4 Mag Ratio** |
|---|---|---|---|---|---|---|
| Patient 2 | Baseline | Y | 2.81 | 0.61 | 4.6 | 12.9 |
| Patient 7 | Baseline | Y | 1.79 | 0.24 | 7.46 | 13.3 |
| Patient 1 | Baseline | N | 0.45 | 1.73 | 0.26 | 0.11 |
| Patient 8 | Baseline | N | 0.73 | 1.45 | 0.50 | 0.36 |
| Patient 6 | Baseline | N | 1.00 | 1.3 | 0.77 | 0.77 |
| Patient 3 | Baseline | N | 0.16 | 0.4 | 0.4 | 0.06 |
| Patient 5 | Baseline | N | 1.17 | 0.24 | 4.8 | 5.61 |

**Example 3 shows the use of IFNγ and IL-6 Indexes.**

Table 4 below shows the calculation of an IFNγ index and an IL-6 index. Patients with a higher IFNγ index values required surgery. As above, patients with a higher ratio or mag ratio required surgery.

**Table 4. IFNγ and IL-6 Indexes.**

| Subject ID | Colectomy | IFNγ + IL-4 + IL-12p40 (IFNγ index) | IL-6 + IL-8 (IL-6 index) | IFNγ Ratio* | IFNγ Mag Ratio ** |
|---|---|---|---|---|---|
| Patient 2 | Y | 2.90 | 0.61 | 4.7 | 13.6 |
| Patient 7 | Y | 2.01 | 0.24 | 8.7 | 17.4 |
| Patient 1 | N | 0.52 | 1.73 | 0.3 | 0.2 |
| Patient 8 | N | 1.73 | 1.45 | 1.2 | 2.0 |
| Patient 6 | N | 1.21 | 1.3 | 0.9 | 1.1 |
| Patient 3 | N | 0.19 | 0.4 | 0.5 | 0.09 |
| Patient 5 | N | 1.24 | 0.25 | 7.0 | 8.7 |

The IFNγ index is defined as the sum of the IFNγ level and the IL-4 level and the IL-12p40 level.
The IL-6 index is defined as the sum of the IL-6 level and the IL-8 level.
Table 4 also includes the "IFNγ ratio" in column 6 that is the quotient of the IFNγ index and the IL-6 index (*e.g.*, IFNγ index /IL-6 index).
Table 4 also includes the "IFNγ mag ratio" in column 7 that is the product of the IL-4 index multiplied by the ratio. In general, higher IFNγ mag ratios require surgery.

**Example 4 tabulates data.**

Table 5 below is a tabulation of the Diagnostic Marker summary. Two (2) individuals (patient 2 and patient 7) required colectomies and 5 patients did not receive colectomies.
Table 5 includes time points at baseline, and 24 hours. The bottom of the table includes the baseline values divided by the 24 hour values.

**Table 5. Index Values at Baseline and 24 hours After Administrations of anti-TNFα Therapy**

| | Subject ID | Timepoint | Colectomy | IFNγ + IL-4+IL-12p40 | IFNγ+IL-4+IL-12p40 | IFNγ+ IL-4 | IL-6+ IL-8 | MAdCAM-1 stability |
|---|---|---|---|---|---|---|---|---|
| baseline | Patient 2 | Baseline | Y | 2.88 | 1.96 | 1.87 | 0.61 | 1.74 |
| | Patient 7 | Baseline | Y | 2.27 | 1.62 | 1.4 | 0.23 | 1.3 |
| | Patient 1 | Baseline | N | 1.18 | 0.35 | 0.28 | 1.73 | 1.06 |
| | Patient 8 | Baseline | N | 0.97 | 0.64 | 0.61 | 1.44 | 0.21 |
| | Patient 6 | Baseline | N | 1.31 | 1.07 | 1 | 1.3 | 0.04 |
| | Patient 3 | Baseline | N | 1.38 | 0.38 | 0.16 | 0.4 | 0.1 |
| | Patient 5 | Baseline | N | 1.92 | 1.69 | 0.69 | 0.24 | 0.35 |
| | | | | | | | | |

(continued)

| | Subject ID | Timepoint | Colectomy | IFNγ + IL-4+IL-12p40 | IFNγ+IL-4+IL-12p40 | IFNγ+ IL-4 | IL-6+ IL-8 | MAdCAM-1 stability |
|---|---|---|---|---|---|---|---|---|
| 24 hours | Patient 2 | Baseline | Y | 1.57 | 0.59 | 0.56 | 1.62 | 1.72 |
| | Patient 7 | Baseline | Y | 1.98 | 0.98 | 0.17 | 0.32 | 1.32 |
| | Patient 1 | Baseline | N | 1.82 | 1.22 | 0.22 | 0.37 | 1.04 |
| | Patient 8 | Baseline | N | 2.83 | 1.95 | 1.27 | 0.17 | 0.32 |
| | Patient 6 | Baseline | N | 2.13 | 1.4 | 1.29 | 1.19 | 0.05 |
| | Patient 3 | Baseline | N | 1.46 | 0.74 | 0.23 | 0.64 | 0.37 |
| | Patient 5 | Baseline | N | 0.93 | 0.34 | 0.23 | 1.09 | 0 |
| | | | | | | | | |
| baseline/ 24 hours | Patient 2 | Baseline | Y | 1.8 | 3.3 | 3.3 | 0.4 | 1.0 |
| | Patient 7 | Baseline | Y | 1.1 | 1.7 | 8.2 | 0.7 | 1.0 |
| | Patient 1 | Baseline | N | 0.6 | 0.3 | 1.3 | 4.7 | 1.0 |
| | Patient 8 | Baseline | N | 0.3 | 0.3 | 0.5 | 8.5 | 0.7 |
| | Patient 6 | Baseline | N | 0.6 | 0.8 | 0.8 | 1.1 | 0.8 |
| | Patient 3 | Baseline | N | 0.9 | 0.5 | 0.7 | 0.6 | 0.3 |
| | Patient 5 | Baseline | N | 2.1 | 5.0 | 3.0 | 0.2 | NA |
| MadCam_Stability = Absolute(Value - Median) / StDev | | | | | | | | |

Table 6 is a tabulation of the Diagnostic Marker Raw data summary. The data table has data points at various times ranging from baseline, 24 hours after initiation of infliximab, 48 hours, 72 hours and 1 week and 2 weeks for some individuals.

Table 6. Diagnostic Marker Raw Data

| Subject ID | Timepoint | Colectomy | TNF$\alpha$ | IFN-Y | IL-4 | IL-6 | IL-8 | IL-12p40 | MAdCAM-1 | IL-10 |
|---|---|---|---|---|---|---|---|---|---|---|
| Patient 1 | Baseline | N | 7.16 | 2.12 | 0.09 | 4.08 | 38.55 | 8.65 | 53178 | 2.83 |
| Patient 1 | 24hr | N | 0.71 | 10.70 | 0.05 | 2.72 | 19.12 | 174.28 | 54670 | 0.40 |
| Patient 1 | 72hr | N | 4.16 | 1.60 | 0.12 | 2.56 | 12.72 | 13.72 | 67978 | 3.11 |
| Patient 1 | 48hr | N | 1.03 | 15.58 | 0.20 | 0.65 | 10.05 | 129.03 | 56054 | 0.62 |
| Patient 2 | Baseline | Y | 7.91 | 18.37 | 0.48 | 1.73 | 11.54 | 11.79 | 195936 | 24.26 |
| Patient 2 | 24hr | Y | 1.17 | 14.48 | 0.30 | 15.21 | 60.83 | 5.27 | 194394 | 2.56 |
| Patient 2 | 72hr | Y | 2.75 | 4.17 | 0.17 | 1.81 | 16.27 | 10.89 | 135074 | 0.83 |
| Patient 2 | 1 week | Y | 4.35 | 3.12 | 0.02 | 0.52 | 8.45 | 14.06 | 182486 | 0.89 |
| Patient 2 | 2 week | Y | 9.34 | 126.19 | 0.48 | 2.59 | 9.95 | 45.66 | 122262 | 31.63 |
| Patient 2 | 48hr | Y | | 10.87 | 0.09 | 2.69 | 20.98 | 213.21 | 155234 | 0.85 |
| Patient 3 | Baseline | N | 8.58 | 3.46 | 0.00 | 0.98 | 8.74 | 4.39 | 112060 | 2.96 |
| Patient 3 | 24hr | N | 0.85 | 8.03 | 0.09 | 4.13 | 35.99 | 89.06 | 126080 | 1.08 |
| Patient 3 | 72hr | N | 0.58 | 1.15 | 0.02 | 0.56 | 7.58 | 13.32 | 133580 | 1.19 |
| Patient 3 | 1 week | N | 1.28 | 21.85 | 0.22 | 0.95 | 9.32 | 23.91 | 115814 | 0.49 |
| Patient 3 | 2 week | N | 3.95 | 0.95 | 0.18 | 1.71 | 13.94 | 5.42 | 112550 | 2.72 |
| Patient 3 | 48hr | N | 0.78 | 1.13 | 0.07 | 0.61 | 5.99 | 8.18 | 132978 | 3.85 |
| Patient 5 | Baseline | N | 1.97 | 3.58 | 0.25 | 0.55 | 5.62 | 9.81 | 124868 | 1.50 |
| Patient 5 | 24hr | N | 0.70 | 6.77 | 0.12 | 1.44 | 98.05 | 19.04 | 107464 | 1.69 |
| Patient 5 | 72hr | N | 0.54 | 9.31 | 0.37 | 2.28 | 28.60 | 209.18 | 118894 | 0.41 |
| Patient 5 | 1 week | N | 0.42 | 3.44 | 0.24 | 1.81 | 17.72 | 57.02 | 154300 | 1.07 |
| Patient 5 | 2 week | N | 0.62 | 6.32 | 0.70 | 1.52 | 4.98 | 10.63 | 114242 | 2.18 |
| Patient 5 | 48hr | N | 0.48 | 12.09 | 0.28 | 1.23 | 5.28 | 19.05 | 114082 | 0.20 |
| Patient 6 | Baseline | N | 2.02 | 21.07 | 0.00 | 5.63 | 11.54 | 27.97 | 105392 | 1.72 |
| Patient 6 | 24hr | N | 0.87 | 17.78 | 0.91 | 2.86 | 27235.13 | 20.59 | 104734 | 0.50 |
| Patient 6 | 72hr | N | 1.17 | 2.21 | 0.31 | 0.50 | 22.54 | 35.02 | 97968 | 1.57 |

(continued)

| Subject ID | Timepoint | Colectomy | TNF$\alpha$ | IFN-Y | IL-4 | IL-6 | IL-8 | IL-12p40 | MAdCAM-1 | IL-10 |
|---|---|---|---|---|---|---|---|---|---|---|
| Patient 6 | 1 week | N | 2.00 | 15.01 | 2.62 | 135.38 | 27235.13 | 20.95 | 109336 | 6.01 |
| Patient 6 | 2 week | N | 3.19 | 266.62 | 0.82 | 32.92 | 56.14 | 13.96 | 103514 | 17.97 |
| Patient 6 | 48hr | N | | 3.18 | 0.43 | 0.99 | 16.64 | 209.62 | 99268 | 0.15 |
| Patient 7 | Baseline | Y | 5.58 | 20.73 | 0.20 | 0.42 | 6.12 | 29.08 | 40756 | 0.08 |
| Patient 7 | 24hr | Y | 1.19 | 3.40 | 0.10 | 1.33 | 23.27 | 141.75 | 40548 | 0.57 |
| Patient 7 | 72hr | Y | 2.22 | 6.75 | 0.66 | 2.02 | 5.33 | 11.51 | 44222 | 1.86 |
| Patient 7 | 1 week | Y | 8.29 | 27.22 | 0.41 | 4.62 | 14.41 | 11.91 | 52364 | 15.43 |
| Patient 7 | 48hr | Y | 2.18 | 2.33 | 0.15 | 0.43 | 24.41 | 38.53 | 43938 | 1.17 |
| Patient 8 | Baseline | N | 2.85 | 10.15 | 0.06 | 4.60 | 24.15 | 131.23 | 118116 | 0.39 |
| Patient 8 | 24hr | N | 1.04 | 62.39 | 0.24 | 1.47 | 7.47 | 119.39 | 123386 | 0.63 |
| Patient 8 | 2 weeks | N | 4.02 | 29.68 | 0.47 | 3.31 | 13.64 | 15.54 | 111480 | 25.47 |
| Patient 8 | 48hr | N | 1.05 | 9.81 | 0.64 | 6.74 | 35.30 | 17.10 | 105600 | 1.72 |

**Example 5 provides cluster analysis.**

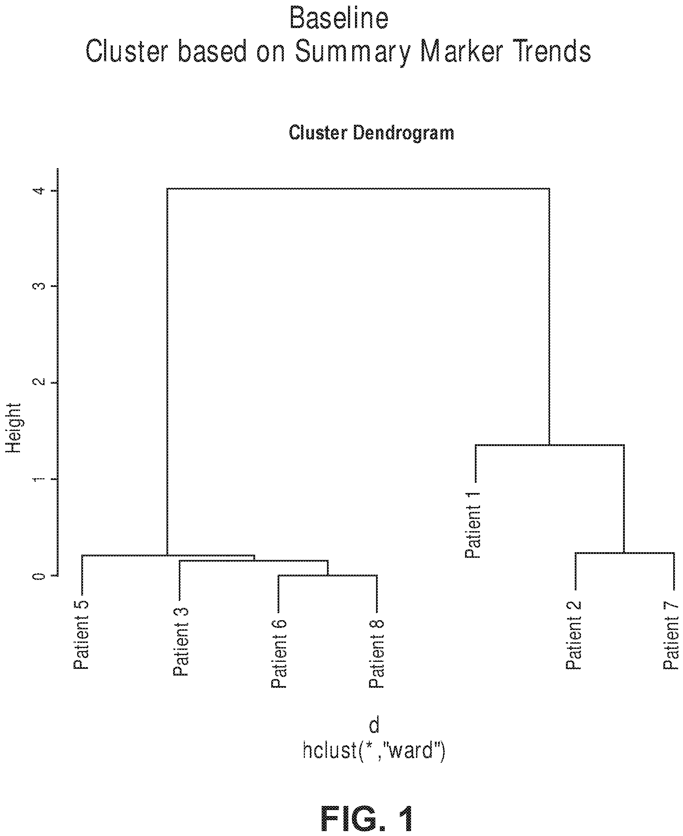

FIG. 1 represents a cluster analysis using the data in Table 5. Table 5 is a tabulation of the Diagnostic Marker summary. Two (2) individuals (patient 2 and patient 7) required colectomies and 5 patients did not receive colectomies. The marker profile for patients requiring a colectomy clustered into distinct classifications.

The cluster analysis in FIG. 1 shows that the data in columns 5-9 at baseline (BL), 24 hrs and BL/24 hrs cluster into two distinct classifications, the left-hand class having the responder cohort and the right hand class having the non-responding cohort. Using these data, it is possible to predict the outcome for therapy.

**Example 6. Various Biomarker Correlations and Associations To Requiring Colectomy.**

This example analyzed a cohort of 8 patients. In this example, 5 subjects did not receive colostomies (the responder cohort) whereas 3 subjects received colectomies (the non-responder cohort). The biomarkers available are as follows: IFNγ, IL-β, IL-2, IL-4, IL-6 IL-8 IL-10, IL-13, GM-CSF, IL-12p40, integrin alpha4beta7, MAdCAM-1, TNFα, IFX, and ATI. The samples were analyzed at the following time points: baseline; 24hrs; 48hrs; 72hrs 1 week; and 2 week.

The data indicate that in certain instances, the following are predictive markers for a colectomy in a subject having UC.

A. Interferon-γ (IFNγ)

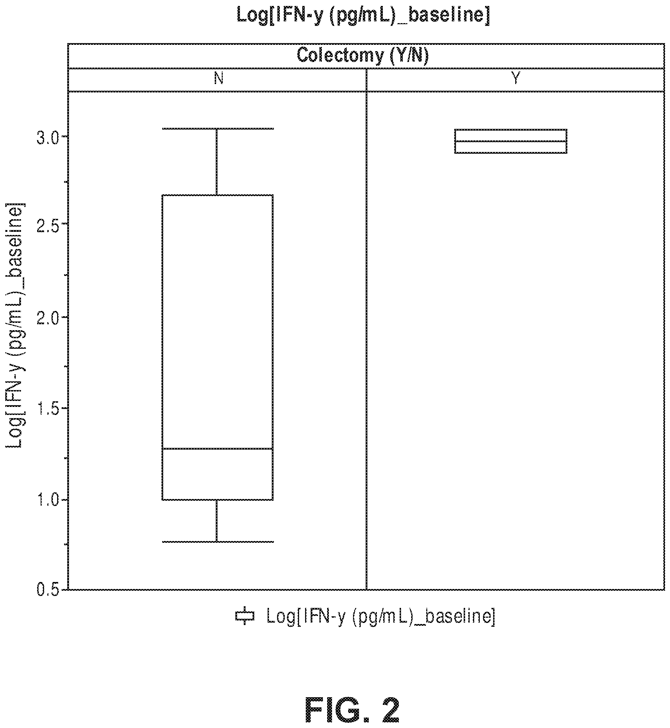

For example, IFNγ at baseline is correlative to a subject requiring a colectomy. FIG. 2 shows a correlation between IFNγ at baseline and colectomy. The t-test p-value was 0.0399, which is significant.

B. Tumor Necrosis Factor alpha (TNFα)

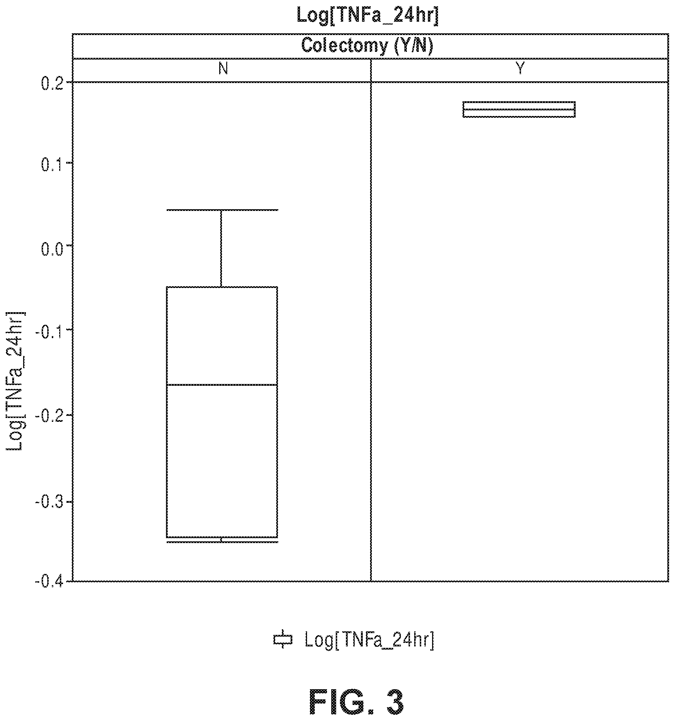

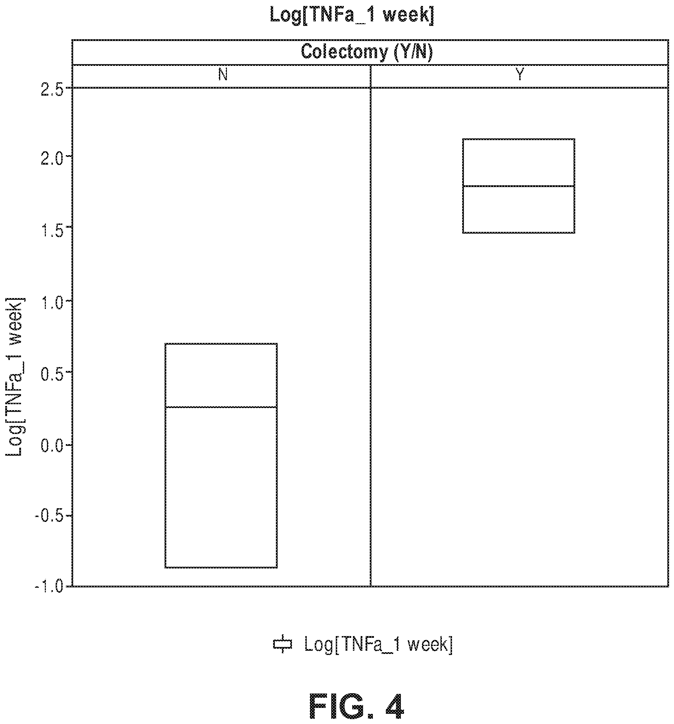

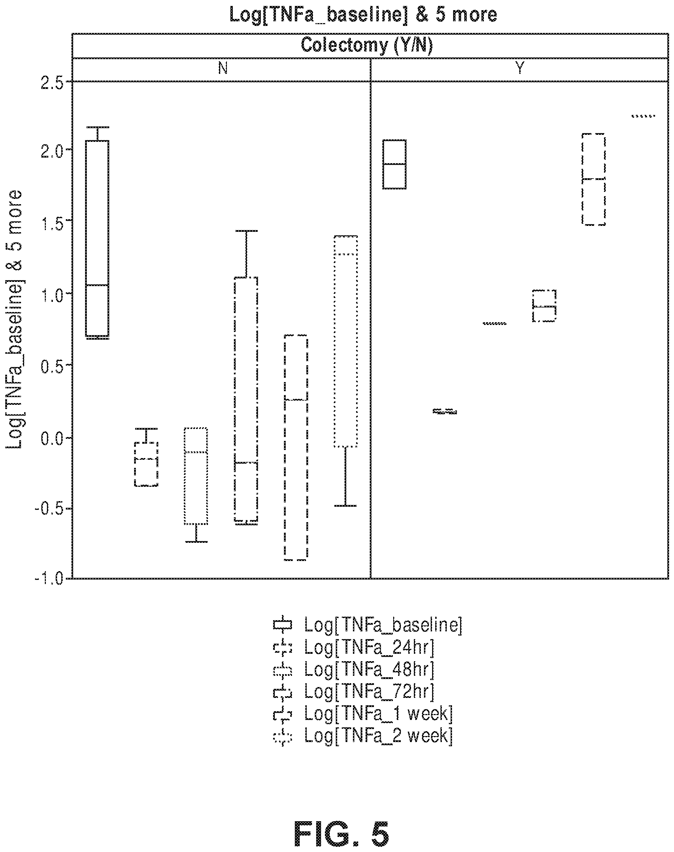

Turning now to FIG. 3, there is a correlation between elevated TNFα levels at 24 hr and a colectomy. The t-test p-value was 0.0079. There was also a correlation of elevated TNFα levels at 1 week are associated with colectomy. FIG. 4 shows that elevated TNFα levels at 1 week are associated with colectomy (p=0.0523). FIG. 5 shows TNFα change in levels over time stratified by colectomy. Higher TNFα levels are indicative of colectomy.

C. IL-8

The data shows a trend that elevated IL-8 levels at 48 hours are associated with a colectomy. The t-test p-value was 0.11. The data further shows the IL-8 level change over time stratified by colectomy.

D. IL-12p40

The data shows a correlation between IL-12p40 at 72 hrs and colectomy. The data shows a change in IL-12p40 levels over time stratified by colectomy (Y/N). Table 7 provides a summary of the results.

Table 7. Data Summary

| Phenotype | Biomarker | Data Set-1 | Data Set-2 |
|---|---|---|---|
| Response | IL-8 | Significant | Trend |
| | IL-12p40 | Significant | |
| | IFNγ | | Significant |
| | TNFα | | Significant |
| ATI | IL-8 | Significant | |
| | IFX | Significant | |

**Example 7. Use of Serum Drug and Biomarker Levels for Predicting Clinical Outcome After High Dose Infliximab (IFX) Treatment of Severe Ulcerative Colitis.**

**Background.** Less than 40% of hospitalized patients with severe ulcerative colitis (UC) treated with infliximab (IFX) respond and avoid colectomy. Patients who receive 10 mg/kg of IFX may have a reduced risk of colectomy as pharmacokinetics are affected by increased tumor necrosis factor-alpha (TNFα) and hypoalbuminemia, which are associated with low IFX concentrations and worse clinical outcomes. The aim of this study is to evaluate whether early serum IFX, TNFα, and inflammatory biomarker levels in hospitalized patients with severe UC treated with high-dose IFX are predictive of clinical response (avoidance of colectomy) and can be used to guide therapy.

Methods. In the study, 11 patients age 18 and over admitted with severe UC (UCDAI ≥ 6, Mayo endoscopic subscore ≥ 2) were enrolled. Table 8 provides a summary of the patient cohort of this study. Patients were excluded if they had other IBD (*i.e.*, Crohn's, indeterminate colitis), prior IFX treatment, or contraindication to anti-TNFa therapy. Patients may be on immunomodulators or have prior exposure to other biologic therapy. Patients had flexible sigmoidoscopy with biopsy for CMV, stool studies for *C. difficile* and bacterial infection, screening for tuberculosis and hepatitis B, and were started on IV corticosteroids. All subjects received a first induction infusion of IFX at a dose of 10 mg/kg. Pre-infusion TNFα and biomarker (IFNγ, IL-1β, IL-2, IL-4, IL-6 IL-8 IL-10, IL-13, GM-CSF, IL-12p40, integrin alpha4beta7, and MAdCAM-1) levels were measured. Serum TNFα, biomarkers, and IFX levels were measured 24, 48 and 72 hours and 1 and 2 weeks after initial infusion.

**Results.** 11 patients were enrolled - 7 males and 4 females. The average age was 41 years old and the average disease duration was 6 years. Three patients required colectomy (27%). The average baseline albumin was 2.8 g/dL overall and 2.9 g/dL among subjects that had colectomy.

Subjects who had colectomy tended to have higher baseline IFNγ and TNFα levels at 24 hours and 1 week, and higher IL-8 at 48 hours (Table 8). These subjects also had lower IL-12p40 levels at 72 hours than those who did not receive colectomy.

**Conclusions.** This pilot study shows biomarker levels among patients with severe UC before and after high dose (10 mg/kg) IFX. The results show that biomarker levels in combination with anti-TNFa levels are predictive of clinical outcome in patients with ulcerative colitis.

**Table 8. Subject Characteristics**

| Subject ID | Sex | Age | Race | Age at Diagnosis | Extent of Disease | Pre IFX Mayo Score | Pre IFX Albumin | Colectomy? Y/N |
|---|---|---|---|---|---|---|---|---|
| 001 | M | 48 | W | 28 | Pancolitis | 11 | 2.6 | N |
| 002 | M | 32 | W | 31 | Pancolitis | 11 | 3.5 | Y |
| 003 | F | 18 | W | 17 | Pancolitis | 11 | 2.2 | N |
| 004 | F | 39 | W | 21 | Left side | 11 | 3.4 | Y |
| 005 | M | 41 | H/L | 41 | Left side | 11 | 3.5 | N |
| 006 | M | 62 | W | 60 | Pancolitis | 11 | 3.1 (1wk post) | N |
| 007 | M | 57 | W | 55 | Left side | 11 | 1.8 | Y |
| 008 | F | 38 | W | 26 | Left side | 11 | 4.3 | N |
| 009 | F | 36 | H/L | 23 | Pancolitis | 11 | 1.8 | N |
| 010 | M | 45 | A | 45 | Pancolitis | 11 | 1.7 | N |
| 011 | M | 38 | W | 37 | Left side | 11 | 3.1 | N |

**Example 8. Serum Drug and TNFα Levels for Predicting Clinical Outcome After High Dose Infliximab (IFX) Treatment of Severe Ulcerative Colitis.**

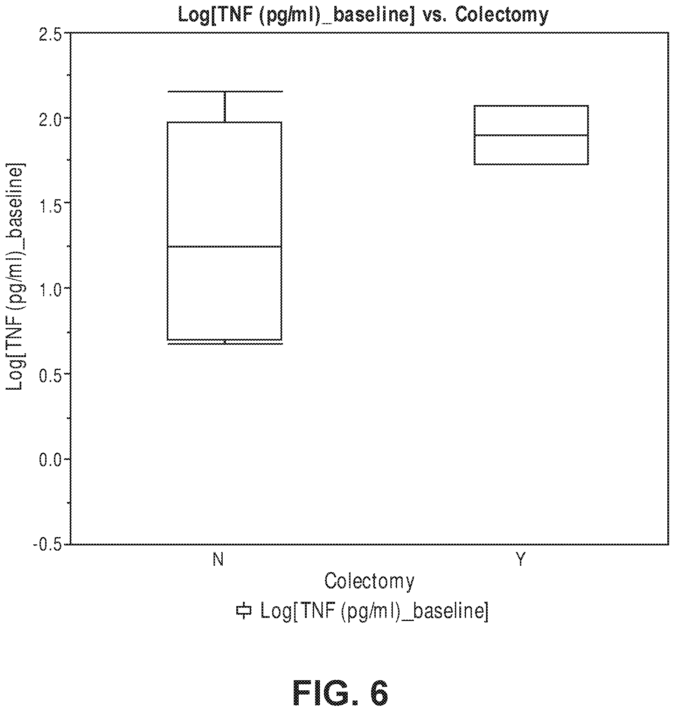

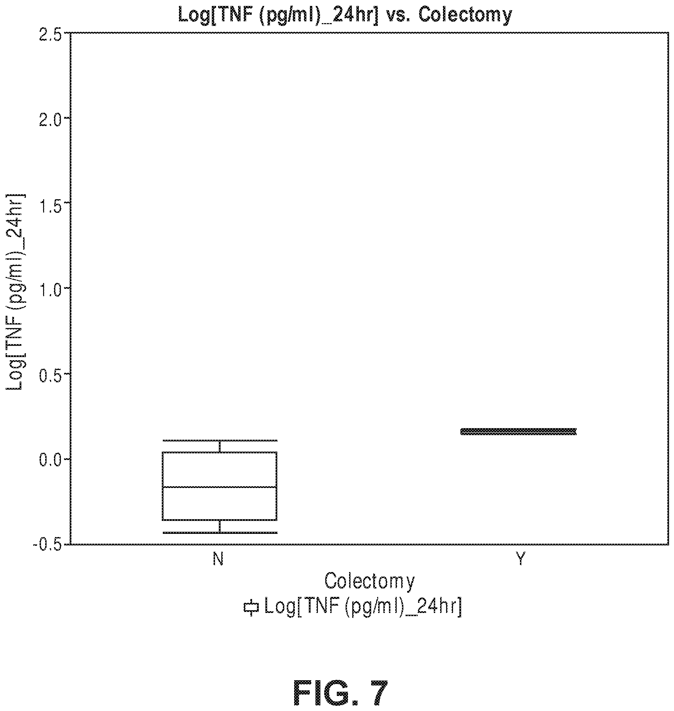

In this study, the patient cohort described in Example 7 was further evaluated. The methods were similar as those described above with the following modifications. Serum IFX and TNFα levels were measured at baseline and at 24 hours, 48 hours, 72 hours, 1 week and 2 weeks after initial infusion of 10 mg/kg of IFX. MAdCAM-1 (mucosal vascular adressin cell adhesion molecule 1) levels were also measured as an assay control. Albumin levels were also measured. FIG. 6 shows that the level of TNFα at baseline in patients who received a colectomy and those who did not receive surgery. FIG. 7 shows that TNFα levels 24 hours post-infusion were higher in patients who received a colectomy vs. those who did not receive surgery.

Median IFX concentration was similar between the patients requiring colectomy and those who did not require colectomy. Patients who received colectomies had higher TNFα concentrations at 24 hours post-infusion (p-value = 0.027). The concentration of MAdCAM-1 was similar and unchanged between the two groups. The data showed that there was no correlation between baseline albumin levels and the rate of colectomy.

**Conclusion.** The level of at least one inflammatory biomarker, and the level of infliximab, and optionally, the level of TNFα are indicative of clinical outcome after IFX treatment of patients with severe UC.

**Example 9. Mag Ratios are Correlative to Ulcerative Colitis Colonscopic Index of Severity (UCCIS) Scores.**

Another endoscopic scoring system is known as the Ulcerative Colitis Colonoscopic Index of Severity (UCCIS). In this scoring system, vascular pattern, ulcerations, bleeding-friability, and granularity are good-to-excellent in predicting overall endoscopic severity and these components are used to make the UCCIS.

The "mag ratio" in column 7 of Table 4 is the product of the TNFα index multiplied by the TNFα ratio. In general, higher mag ratios require surgery. Patients with high TNFα mag ratios have high UCCIS scores (24-34). A UCCIS score is a numerical score derived by entering the individual grade of four descriptors into a formula:

$$(3.1 \text{ x vascular pattern}) + (3.6 \text{ x granularity}) + (3.5 \text{ x ulceration}) + (2.5 \text{ x bleeding/friability}).$$

Patients with a normal vascular pattern are given a score of 0, while those with a partial loss of pattern were given a 1, and patients with complete obliteration of vascular pattern are given a 2. Granularity is divided into 0-3, with 0 corresponding with no granularity, 1 with fine granularity, and 2 with coarse granularity . Ulcerations is graded as absent (0), erosions or pinpoint ulcers (1), multiple shallow ulcers with mucus (2), deep ulcers (3), or diffuse ulcers involving more than 30% of the mucosa (4). In terms of bleeding and friability, mucosa with no bleeding or friability is designated 0, while mucosa with friability and bleeding with minimal touch is rated 1, and tissue with spontaneous bleeding is given 2. (See, *e.g.*, Thia KT, Inflamm Bowel Dis, 2011, 17:1257-64).

Patients with high UCCIS scores (24-34) have high TNFα mag ratios (5-12). In other words, UCCIS scores are correlative to TNFα mag ratios. The scores can also be correlated to IL-4 mag ratios.

**Claims**

1. A method for predicting a clinical outcome of a subject having ulcerative colitis (UC), the method comprising:

   (a) determining a prognostic marker profile by detecting the presence or level of at least one prognostic marker, which is a cytokine selected from the group IFNγ, IL-4, IL-12p40, and a combination thereof in a sample from the subject;
   (b) calculating one or more of an IL-4 index value, which is based on a sum of the level of IFNγ and the level of IL-4 in the sample or an IFNγ index value, which is based on the sum of IFNγ, IL-4 and IL-12p40; and
   (c) classifying the subject having UC as either a responder or a non-responder to an anti-TNFα drug therapy.

2. The method of claim **1**, determining the prognostic marker profile based on the IL-4 index value.

3. The method of claim **2**, wherein the subject is classified as a non-responder if the IL-4 index value is higher compared to a corresponding UC average IL-4 index value.

4. The method of claim **2**, wherein the subject is classified as a responder if the IL-4 index value is equal or lower compared to the corresponding UC average IL-4 index value.

5. The method of claim **2**, wherein determining the prognostic marker profile is based on the IFNγ index value.

6. The method of claim **5**, wherein the subject is classified as a non-responder if the IFNγ index value is higher compared to a corresponding UC average IFNγ index value.

7. The method of claim **5**, wherein the subject is classified as a responder if the IFNγ index value is equal or lower compared to the corresponding UC average IFNγ index value.

8. The method of claim **1**, wherein the sample is selected from the group consisting of whole blood, plasma, serum, urine, saliva, or other bodily fluids.

9. The method of claim **1**, determining the prognostic marker profile is based on the IL-4 index value and the IFNγ index value.

**Patentansprüche**

1. Verfahren zur Vorhersage eines klinischen Ergebnisses eines Individuums mit Colitis ulcerosa (CU), wobei das Verfahren Folgendes umfasst:

(a) Bestimmen eines prognostischen Markerprofils durch Nachweis der Anwesenheit oder des Spiegels mindestens eines prognostischen Markers, der ein Cytokin ist, das aus der Gruppe IFNγ, IL-4, IL-12p40 und einer Kombination davon in einer Probe von dem Individuum ausgewählt wird;
(b) Berechnen eines oder mehrerer eines IL-4-Indexwerts, der auf einer Summe des Spiegels von IFNγ und des Spiegels von IL-4 in der Probe basiert, oder eines IFNγ-Indexwerts, der auf der Summe aus IFNγ, IL-4 und IL-12p40 basiert; und
(c) Klassifizieren des Individuums mit CU entweder als Responder oder Non-Responder auf eine Anti-TNFα-Arzneimitteltherapie.

2. Verfahren nach Anspruch **1**, wobei das prognostische Markerprofil basierend auf dem IL-4-Indexwert bestimmt wird.

3. Verfahren nach Anspruch **2**, wobei das Individuum als Non-Responder klassifiziert wird, wenn der IL-4-Indexwert im Vergleich zu einem entsprechenden CU-Durchschnitts-IL-4-Indexwert höher ist.

4. Verfahren nach Anspruch **2**, wobei das Individuum als Responder klassifiziert wird, wenn der IL-4-Indexwert im Vergleich zu dem entsprechenden CU-Durchschnitts-IL-4-Indexwert gleich oder niedriger ist.

5. Verfahren nach Anspruch **2**, wobei das Bestimmen des prognostischen Markerprofils auf dem IFNγ-Indexwert basiert.

6. Verfahren nach Anspruch **5**, wobei das Individuum als Non-Responder klassifiziert wird, wenn der IFNγ-Indexwert im Vergleich zu einem entsprechenden CU-Durchschnitts-IFNγ-Indexwert höher ist.

7. Verfahren nach Anspruch **5**, wobei das Individuum als Responder klassifiziert wird, wenn der IFNγ-Indexwert im Vergleich zu dem entsprechenden CU-Durchschnitts-IFNγ-Indexwert gleich oder niedriger ist.

8. Verfahren nach Anspruch **1**, wobei die Probe ausgewählt ist aus der Gruppe bestehend aus Vollblut, Plasma, Serum, Urin, Speichel oder anderen Körperflüssigkeiten.

9. Verfahren nach Anspruch **1**, wobei das Bestimmen des prognostischen Markerprofils auf dem IL-4-Indexwert und dem IFNγ-Indexwert basiert.

**Revendications**

1. Procédé pour prédire une issue clinique d'un sujet présentant une rectocolite hémorragique (UC), le procédé comprenant :

(a) la détermination d'un profil de marqueur de pronostic en détectant la présence ou le niveau d'au moins un marqueur de pronostic, qui est une cytokine choisie dans le groupe IFNγ, IL-4, IL-12p40, et une combinaison de celles-ci dans un échantillon provenant du sujet ;
(b) le calcul d'une ou plusieurs parmi une valeur d'indice d'IL-4, qui est basée sur une somme du taux d'IFNγ et du taux d'IL-4 dans l'échantillon ou une valeur d'indice d'IFNγ, qui est basée sur la somme d'IFNγ, d'IL-4 et d'IL-12p40 ; et
(c) la classification du sujet présentant une UC soit comme étant répondant, soit comme étant non-répondant à une pharmacothérapie anti-TNFa.

2. Procédé selon la revendication **1**, déterminant le profil de marqueur de pronostic sur la base de la valeur d'indice d'IL-4.

**3.** Procédé selon la revendication **2**, dans lequel le sujet est classé comme non-répondant si la valeur d'indice d'IL-4 est plus élevée par comparaison avec une valeur d'indice d'IL-4 moyenne d'UC correspondante.

**4.** Procédé selon la revendication **2**, dans lequel le sujet est classé comme non-répondant si la valeur d'indice d'IL-4 est égale ou moins élevée par comparaison avec la valeur d'indice d'IL-4 moyenne d'UC correspondante.

**5.** Procédé selon la revendication **2**, dans lequel la détermination du profil de marqueur de pronostic est basée sur la valeur d'indice d'IFNγ.

**6.** Procédé selon la revendication 5, dans lequel le sujet est classé comme non-répondant si la valeur d'indice d'IFNγ est plus élevée par comparaison avec une valeur d'indice d'IFNγ moyenne d'UC correspondante.

**7.** Procédé selon la revendication 5, dans lequel le sujet est classé comme non-répondant si la valeur d'indice d'IFNγ est égale ou moins élevée par comparaison avec la valeur d'indice d'IFNγ moyenne d'UC correspondante.

**8.** Procédé selon la revendication **1**, dans lequel l'échantillon est choisi dans le groupe constitué de sang total, plasma, sérum, urine, salive, ou d'autres fluides corporels.

**9.** Procédé selon la revendication **1**, la détermination du profil de marqueur de pronostic est basée sur la valeur d'indice d'IL-4 et la valeur d'indice d'IFNγ.

Baseline
Cluster based on Summary Marker Trends

FIG. 1

FIG. 2

FIG. 3

Log[TNFa_1 week]

FIG. 4

Log[TNFa_baseline] & 5 more

FIG. 5

FIG. 6

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8574855 B
- US 20140051184 A
- US 2013029685 A

### Non-patent literature cited in the description

- **TOEDTER et al.** *Am J. Gastroenterol.,* 2011, vol. 106, 1272-1280
- **SANDBORN.** *Gastroenterology,* 2014, vol. 146, 96-109
- **DINARELLO.** *Eur J Immunol,* 2007, vol. 37 (1), S34-S45
- **NEURATH.** *Nat Rev Immunol,* 2014, vol. 14, 329-342
- **STROBER ; FUSS.** *Gastroenterology,* 2011, vol. 140 (6), 1756-1767
- **JONES et al.** *Nature,* 1989, vol. 338, 225-228
- **PENNICA, D. et al.** *Nature,* 1984, vol. 312, 724
- **HASHIDA et al.** *J. Clin. Lab. Anal.,* 1997, vol. 11, 267-86
- **PAINE.** *Gastroenterol,* 2014, vol. 2 (2), 161-168
- **TRAVIS et al.** *Gut,* 2012, vol. 61, 535-42
- **THIA KT.** *Inflamm Bowel Dis,* 2011, vol. 17, 1257-64
- **SCHROEDER.** *N Engl J Med,* 1987, vol. 317, 1625-29
- **BREIMAN.** *Machine Learning,* 2001, vol. 45, 5-32
- **BREIMAN et al.** Classification and Regression Trees. Chapman and Hall, 1984
- **STEINBERG et al.** CART: Tree-Structured Non-Parametric Data Analysis. *Salford Systems,* 1995
- **FREEMAN et al.** Neural Networks: Algorithms, Applications and Programming Techniques. Addison-Wesley Publishing Company, 1991
- **ZADEH.** *Information and Control,* 1965, vol. 8, 338-353
- **ZADEH.** IEEE Trans. on Systems, Man and Cybernetics. 1973, vol. 3, 28-44
- **GERSHO et al.** Vector Quantization and Signal Compression. Kluywer Academic Publishers, 1992
- **CRISTIANINI et al.** An Introduction to Support Vector Machines and Other Kernel-Based Learning Methods. Cambridge University Press, 2000